# EUROPEAN PATENT APPLICATION

(11) **EP 4 813 382 A1**
(43) Date of publication of application: **30.09.2026**
(21) Application number: 25187957.3
(22) Date of filing: 07.07.2025
(51) Int. Cl.: A61K 31/40, A61K 31/4015, A61P 25/28

(54) **(S)-3-(2,3-DIFLUOROPHENYL)-3-METHOXYPYRROLIDINE AT AN APPROPRIATE DOSAGE FOR USE IN THE TREATMENT OF DISORDERS ASSOCIATED WITH NEURODEGENERATIVE DISEASES**

(30) Priority: 27.03.2025 GB 202504572
(71) Applicant: Integrative Research Laboratories Sweden AB, 413 46 Göteborg (SE)
(72) Inventor: TEDROFF, Joakim, 182 37 Danderyd (SE); WATERS, Nicholas, 413 19 Göteborg (SE); WATERS, Susanna Holm, 413 19 Göteborg (SE); HANSSON, Per Fredrik, 24465 Furulund (SE)
(74) Representative: Potter Clarkson

(57) **Abstract**

There is provided herein the compound (S)-3-(2,3-difluorophenyl)-3-methoxypyrrolidine, or a pharmaceutically acceptable salt thereof, 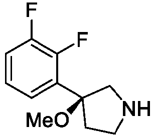 for use in the treatment of disorder associated with neurodegenerative disease or disorder, wherein the treatment comprises administering (S)-3-(2,3-difluorophenyl)-3-methoxypyrrolidine, or a pharmaceutically acceptable salt thereof, to a subject in an amount that results in an appropriate plasma concentration of the active ingredient to provide an effective treatment, as described herein.

## Description

### Field of the Invention

The present invention relates to a new medical use, as well as new metabolites of a known compound that may be used as markers to determine the optimum exposure of an active ingredient. In particular, the present invention relates to the use of the compound (S)-3-(2,3-difluorophenyl)-3-methoxypyrrolidine in the treatment of disorders associated with neurodegenerative diseases, which treatment comprises administering the compound at an appropriate dosage to provide an effective level of exposure to the active ingredient in individual patients. The invention further relates to metabolites of (S)-3-(2,3-difluorophenyl)-3-methoxypyrrolidine that are present in plasma in concentrations that are highly correlated to the exposure to the active ingredient and the use of such metabolites in determining and defining effective doses of (S)-3-(2,3-difluorophenyl)-3-methoxypyrrolidine for individual patients.

### Background of the Invention

The listing or discussion of an apparently prior-published document in this specification should not necessarily be taken as an acknowledgement that the document is part of the state of the art or is common general knowledge.

The compound (S)-3-(2,3-difluorophenyl)-3-methoxypyrrolidine (pirepemat) is in clinical development for the treatment of Parkinson's disease, with the aim to improve postural instability, and therefore reduce the rates of falls, and consequently injuries from falls in Parkinson's patients. However, in the recently reported topline results from the REACT-PD Phase IIb study, a daily dose of 600 mg of pirepemat did not show a statistically significant reduction in the fall rate of Parkinson's patients compared to the placebo.

Given this, there remains a need to develop effective methods for the treatment of Parkinson's disease and the associated postural instability.

### Detailed Description of the Invention

It has now surprisingly been found that certain plasma concentrations of (S)-3-(2,3-difluorophenyl)-3-methoxypyrrolidine allow for reducing the rate of falls in Parkinson's patients. Therefore, the administration of (S)-3-(2,3-difluorophenyl)-3-methoxypyrrolidine, or a pharmaceutically acceptable salt thereof, in an amount resulting in such a plasma concentration provides an effective treatment for disorders associated with neurodegenerative diseases.

The effective absolute dose of the (S)-3-(2,3-difluorophenyl)-3-methoxypyrrolidine varies between patients, but there is a clear relationship between the plasma exposure of the compound and the effectiveness of the treatment. In particular, the relationship between pirepemat exposure and efficacy follows a biphasic pattern, with too high and too low exposures to the drug being ineffective, but intermediate levels of exposure being associated with a significant reduction in fall rate in Parkinson's patients.

Certain metabolites of (S)-3-(2,3-difluorophenyl)-3-methoxypyrrolidine have also surprisingly been found to be highly correlated to the exposure to the active ingredient, and therefore can be used to accurately determine an appropriate dosage for an individual patient.

### New medical use

In a first aspect of the invention, there is provided the compound (S)-3-(2,3-difluorophenyl)-3-methoxypyrrolidine, or a pharmaceutically acceptable salt thereof, 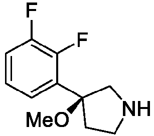 for use in the treatment of a neurodegenerative disease or disorder, wherein the treatment comprises administering (S)-3-(2,3-difluorophenyl)-3-methoxypyrrolidine, or a pharmaceutically acceptable salt thereof, to a subject in an amount that results in:
(i) a minimum plasma concentration (e.g. Cmin or Ctrough) of (S)-3-(2,3-difluorophenyl)-3-methoxypyrrolidine of from about 500 to about 3000 nM, such as during a dosing interval (for example before dosing in the morning); and/or
(ii) a minimum plasma concentration (e.g. Cmin or Ctrough) of 4-(2,3-difluorophenyl)-4-methoxy-2-pyrrolidinone 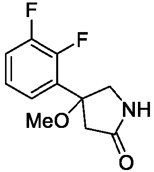
of from about 800 nM to about 3200 nM, such as during a dosing interval (for example before dosing in the morning).

In an alternative first aspect of the invention, there is provided a method of treating a disorder associated with a neurodegenerative disease comprising administering a therapeutically effective amount of the compound (S)-3-(2,3-difluorophenyl)-3-methoxypyrrolidine, or a pharmaceutically acceptable salt thereof, to a subject in need thereof, wherein the therapeutically effective amount of the compound results in:
(i) a minimum plasma concentration (e.g. Cmin or Ctrough) of (S)-3-(2,3-difluorophenyl)-3-methoxypyrrolidine of from about 500 nM to about 3000 nM, such as during a dosing interval (for example before dosing in the morning); and/or
(ii) a minimum plasma concentration (e.g. Cmin or Ctrough) of 4-(2,3-difluorophenyl)-4-methoxy-2-pyrrolidinone of from about 800 nM to about 3200 nM, such as during a dosing interval (for example before dosing in the morning).

In a further alternative first aspect of the invention, there is provided the use of the compound (S)-3-(2,3-difluorophenyl)-3-methoxypyrrolidine, or a pharmaceutically acceptable salt thereof, for the manufacture of a medicament for the treatment of a disorder associated with a neurodegenerative disease, wherein the treatment comprises administering the compound (S)-3-(2,3-difluorophenyl)-3-methoxypyrrolidine, or a pharmaceutically acceptable salt thereof, in an amount that results in:
(i) a minimum plasma concentration (e.g. Cmin or Ctrough) of (S)-3-(2,3-difluorophenyl)-3-methoxypyrrolidine of from about 500 nM to about 3000 nM, such as during a dosing interval (for example before dosing in the morning); and/or
(ii) a minimum plasma concentration (e.g. Cmin or Ctrough) of 4-(2,3-Difluorophenyl)-4-methoxy-2-pyrrolidinone of from about 800 nM to about 3200 nM during a dosing interval (for example before dosing in the morning).

Unless indicated otherwise, all technical and scientific terms used herein will have their common meaning as understood by one of ordinary skill in the art to which this invention pertains.

Pharmaceutically acceptable salts of (S)-3-(2,3-difluorophenyl)-3-methoxypyrrolidine that may be mentioned include acid addition salts. Such salts may be formed by conventional means, for example by reaction of the free base form of the compound with one or more equivalents of an appropriate amount (e.g. 1 or more equivalents) of an appropriate acid or base, optionally in a solvent, or in a medium in which the salt is insoluble, followed by removal of said solvent, or said medium, using standard techniques (e.g. *in vacuo,* by freeze-drying or by filtration). Salts may also be prepared using techniques known to those skilled in the art, such as by exchanging a counter-ion of a compound of the invention in the form of a salt with another counter-ion, for example using a suitable ion exchange resin.

Particular acid addition salts that may be mentioned include those formed by reaction with corresponding acids, thus protonating the compound of the invention, to form carboxylate salts (e.g. formate, acetate, trifluoroacetate, propionate, isobutyrate, heptanoate, decanoate, caprate, caprylate, stearate, acrylate, caproate, propiolate, ascorbate, citrate, glucuronate, glutamate, glycolate, α-hydroxybutyrate, lactate, tartrate, phenylacetate, mandelate, phenylpropionate, phenylbutyrate, benzoate, chlorobenzoate, methylbenzoate, hydroxybenzoate, methoxybenzoate, dinitrobenzoate, o-acetoxy-benzoate, salicylate, nicotinate, isonicotinate, cinnamate, oxalate, malonate, succinate, suberate, sebacate, fumarate, malate, maleate, hydroxymaleate, hippurate, phthalate or terephthalate salts), halide salts (e.g. chloride, bromide or iodide salts), sulphonate salts (e.g. benzenesulphonate, methyl-, bromo- or chloro-benzenesulphonate, xylenesulphonate, methanesulphonate, ethanesulphonate, propanesulphonate, hydroxy-ethanesulphonate, 1- or 2-naphthalene-sulphonate or 1,5-naphthalene-disulphonate salts) or sulphate, pyrosulphate, bisulphate, sulphite, bisulphite, phosphate, monohydrogenphosphate, dihydrogenphosphate, metaphosphate, pyrophosphate or nitrate salts, and the like.

More particular salts that may be mentioned include carboxylic acid salts. Yet more particular salts that may be mentioned include dicarboxylic acid salts and most particularly, the salt is a fumaric acid salt.

Accordingly, in particular embodiments of the uses and methods described herein, the compound (S)-3-(2,3-difluorophenyl)-3-methoxypyrrolidine is used (i.e. administered) in the form of a fumarate salt (e.g. a 1:1 salt of (S)-3-(2,3-difluorophenyl)-3-methoxypyrrolidine:fumaric acid).

The present invention also embraces the use of isotopically-labelled (S)-3-(2,3-difluorophenyl)-3-methoxypyrrolidine, in the compounds and methods described herein. Such isotopically labelled compounds are identical to those recited herein, but for the fact that one or more atoms are replaced by an atom having an atomic mass or mass number different from the atomic mass or mass number usually found in nature (or the most abundant one found in nature). All isotopes of any particular atom or element as specified herein are contemplated within the scope of the compounds of the invention. Hence, the compounds of the invention also include deuterated compounds, i.e. compounds of the invention in which one or more hydrogen atoms are replaced by the hydrogen isotope deuterium.

### 4-(2,3-difluorophenyl)-4-methoxy-2-pyrrolidinone (below) is a metabolite of (S)-3-(2,3-difluorophenyl)-3-methoxypyrrolidine.

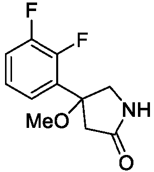

In the methods described herein, the plasma concentration of 4-(2,3-difluorophenyl)-4-methoxy-2-pyrrolidinone was determined using achiral chromatography, and therefore the enantiomeric purity of the metabolite was not assessed. However, it is believed that the compound is present as the single (S)-enantiomer, (S)-4-(2,3-difluorophenyl)-4-methoxy-2-pyrrolidinone as the metabolite is not expected to racemise, or partly racemise, in vivo.

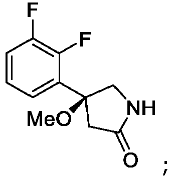

Accordingly, references herein to 4-(2,3-difluorophenyl)-4-methoxy-2-pyrrolidinone and the plasma concentration of this compound may particularly be understood to refer to (S)-4-(2,3-difluorophenyl)-4-methoxy-2-pyrrolidinone. Thus, in particular embodiments, the 4-(2,3-difluorophenyl)-4-methoxy-2-pyrrolidinone is (S)-4-(2,3-difluorophenyl)-4-methoxy-2-pyrrolidinone.

A further metabolite of 4-(2,3-difluorophenyl)-4-hydroxy-2-pyrrolidinone is 4-(2,3-difluorophenyl)-4-hydroxy-2-pyrrolidinone (structure below).

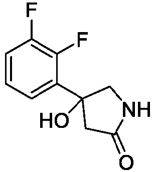

As for 4-(2,3-difluorophenyl)-4-methoxy-2-pyrrolidinone, as the metabolite is not expected to racemise, or partly racemise, in vivo it is believed that it is present as the (S)-enantiomer ((*S*)-4-(2,3-difluorophenyl)-4-hydroxy-2-pyrrolidinone (structure below))

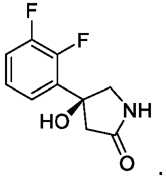

The skilled person will understand that the plasma concentration of the active ingredient and the metabolites of the active ingredient may vary between different individuals even when the different patients have been given the same dosage of the active ingredient. In particular, the ratio between the plasma concentration of the active ingredient and the plasma concentration of the metabolites may vary between individuals. Thus, it is necessary to determine the dose necessary to provide the required plasma concentration for an individual before commencing treatment.

In particular embodiments of the treatments described herein, the treatment comprises the steps of
(i) determining the dose of (S)-3-(2,3-difluorophenyl)-3-methoxypyrrolidine required to result in a plasma concentration of (S)-3-(2,3-difluorophenyl)-3-methoxypyrrolidine and/or 4-(2,3-difluorophenyl)-4-methoxy-2-pyrrolidinone within the effective range for the subject being treated (i.e. a minimum plasma concentration (e.g. Cmin or Ctrough (for example Ctrough)) of (S)-3-(2,3-difluorophenyl)-3-methoxypyrrolidine of from about 500 to about 3000 nM), such as during a dosing interval (for example before dosing in the morning); and/or a minimum plasma concentration (e.g. Cmin or Ctrough (for example Ctrough)) of 4-(2,3-difluorophenyl)-4-methoxy-2-pyrrolidinone (of from about 800 nM to about 3200 nM) during a dosing interval (for example before dosing in the morning)); then
(ii) proceeding with the treatment using the dose so determined.

In particular, the required dose may be determined following a drug titration procedure.

The skilled person will understand that drug titration refers to the process of gradually adjusting the dose of a medication to determine the dose necessary to achieve the desired effect. In a typical titration procedure, the medication is initially administered at a low dose and then slowly increased over time in order to achieve the optimal dose to achieve the required effect. The optimal dose may also be determined by monitoring the patient's plasma concentration at initial dose, and calculating the required dose from this. Thus, the optimal dose may be determined by monitoring the plasma concentration of the active ingredient and/or its metabolite(s) and calculating the dose required to achieve the desirable plasma concentration of the active ingredient and/or its metabolites.

Accordingly, in particular embodiments of the methods and uses described herein, the amount of (S)-3-(2,3-difluorophenyl)-3-methoxypyrrolidine, or a pharmaceutically acceptable salt thereof, required to result in a minimum plasma concentration of (S)-3-(2,3-difluorophenyl)-3-methoxypyrrolidine and/or 4-(2,3-difluorophenyl)-4-methoxy-2-pyrrolidinone ((S)-4-(2,3-difluorophenyl)-4-methoxy-2-pyrrolidinone) (as defined herein) (in an individual patient) is determined by titration.

The skilled person will understand that, in order to measure the minimum plasma concentration of a drug as described herein (e.g. Cmin or Ctrough (for example, Ctrough)), it is necessary to administer the drug for a period of time in order to achieve a steady state plasma concentration, at which point the amount of drug administered in a given time period is equal to the amount of drug eliminated in the same time period. Typically, this period of time may correspond to at least 4 or at least 5 half lives of the drug.

In particular embodiments, the titration comprises the steps of:
(i) administering a first dose of (S)-3-(2,3-difluorophenyl)-3-methoxypyrrolidine, or a pharmaceutically acceptable salt thereof, (for example a dose of from about 25 to about 100 mg (of the active ingredient), e.g. about 50 mg, 1 to 3 times a day (e.g. 3 times)) to a subject during a first time period sufficient to achieve a steady state plasma concentration of (S)-3-(2,3-difluorophenyl)-3-methoxypyrrolidine, 4-(2,3-difluorophenyl)-4-methoxy-2-pyrrolidinone ((S)-4-(2,3-difluorophenyl)-4-methoxy-2-pyrrolidinone) and/or 4-(2,3-difluorophenyl)-4-hydroxy-2-pyrrolidinone ((*S*)-4-(2,3-difluorophenyl)-4-hydroxy-2-pyrrolidinone), which time period may be from about 1 day to about 10 days (e.g from about 1 day to about 7 days);
(ii) measuring the minimum plasma concentration (Cmin or Ctrough (e.g. Ctrough)) of (S)-3-(2,3-difluorophenyl)-3-methoxypyrrolidine, 4-(2,3-difluorophenyl)-4-methoxy-2-pyrrolidinone ((S)-4-(2,3-difluorophenyl)-4-methoxy-2-pyrrolidinone) and/or 4-(2,3-difluorophenyl)-4-hydroxy-2-pyrrolidinone ((*S*)-4-(2,3-difluorophenyl)-4-hydroxy-2-pyrrolidinone) (and, optionally, comparing it to a reference range for the minimum plasma concentration); and
(iii)determining the dose of (S)-3-(2,3-difluorophenyl)-3-methoxypyrrolidine, or a pharmaceutically acceptable salt thereof, required to provide a minimum plasma concentration within the reference range using the plasma concentration of (S)-3-(2,3-difluorophenyl)-3-methoxypyrrolidine and/or 4-(2,3-difluorophenyl)-4-methoxy-2-pyrrolidinone and/or 4-(2,3-difluorophenyl)-4-hydroxy-2-pyrrolidinone ((*S*)-4-(2,3-difluorophenyl)-4-hydroxy-2-pyrrolidinone) measured in step (ii).

In particular embodiments, determining the dose of (S)-3-(2,3-difluorophenyl)-3-methoxypyrrolidine, or a pharmaceutically acceptable salt thereof, required to provide a minimum plasma concentration within the reference range (step (iii)) comprises the steps of:
(iv)administering an increased dose of (S)-3-(2,3-difluorophenyl)-3-methoxypyrrolidine, or a pharmaceutically acceptable salt thereof, for a further time period (for example, the increased dose may be from about 25 to about 100 mg (of the active ingredient), e.g. 50 mg, higher than the previous (first) dose, 1 to 3 times (e.g. 3 times) a day, to the subject for a time period sufficient to achieve a steady state plasma concentration of (S)-3-(2,3-difluorophenyl)-3-methoxypyrrolidine and/or 4-(2,3-difluorophenyl)-4-methoxy-2-pyrrolidinone ((S)-4-(2,3-difluorophenyl)-4-methoxy-2-pyrrolidinone), which time period may be from about 1 day to about 10 days (e.g from about 1 day to about 7 days); and
(v) measuring the minimum plasma concentration (Cmin or Ctrough, (e.g. Ctrough)) of (S)-3-(2,3-difluorophenyl)-3-methoxypyrrolidine, 4-(2,3-difluorophenyl)-4-methoxy-2-pyrrolidinone ((S)-4-(2,3-difluorophenyl)-4-methoxy-2-pyrrolidinone) and/or 4-(2,3-difluorophenyl)-4-hydroxy-2-pyrrolidinone ((*S*)-4-(2,3-difluorophenyl)-4-hydroxy-2-pyrrolidinone); (and, optionally, comparing it to a reference range for the minimum plasma concentration); and
(vi) optionally, repeating steps (iv) and (v), until a plasma concentration within the reference range is achieved.

In particular, the reference ranges for the minimum plasma concentration of (S)-3-(2,3-difluorophenyl)-3-methoxypyrrolidine and/or 4-(2,3-difluorophenyl)-4-methoxy-2-pyrrolidinone ((S)-4-(2,3-difluorophenyl)-4-methoxy-2-pyrrolidinone) correspond to the minimum plasma concentrations defined in the uses and methods defined herein.

In particular embodiments, determining the dose of (S)-3-(2,3-difluorophenyl)-3-methoxypyrrolidine, or a pharmaceutically acceptable salt thereof, required to provide a minimum plasma concentration within the reference range (step (iii)) comprises calculating the dose required from the plasma concentrations of (S)-3-(2,3-difluorophenyl)-3-methoxypyrrolidine and/or 4-(2,3-difluorophenyl)-4-methoxy-2-pyrrolidinone ((S)-4-(2,3-difluorophenyl)-4-methoxy-2-pyrrolidinone) measured in step (ii) and/or, optionally, step (v). For example, the required dose may be calculated on the basis that (S)-3-(2,3-difluorophenyl)-3-methoxypyrrolidine displays dose-proportional (pharmaco)kinetics.

The skilled person will understand that an active ingredient is considered to have dose proportional (pharmaco)kinetics if the drug's exposure (e.g. as measured by plasma concentration, such as Ctrough) in the body increases proportionally (i.e. in linear or approximately linear proportion) to the dose administered.

Accordingly, the required dose of (S)-3-(2,3-difluorophenyl)-3-methoxypyrrolidine, or a pharmaceutically acceptable salt thereof, may be calculated from the (minimum) plasma concentration of (S)-3-(2,3-difluorophenyl)-3-methoxypyrrolidine measured in step (ii), and/or, optionally, step (v) (e.g. step (ii)), by determining the percentage increase in plasma concentration required to reach a plasma concentration within the reference range for (S)-3-(2,3-difluorophenyl)-3-methoxypyrrolidine, and increasing the dose administered by the same percentage.

The calculation of the required dose of (S)-3-(2,3-difluorophenyl)-3-methoxypyrrolidine, or a pharmaceutically acceptable salt thereof, to arrive at the required minimum plasma concentration(s), may also be expressed as the following equation: ${Dose}_{R} = {Dose}_{A} \times \left(\frac{Plasma conc {.}_{R}}{Plasma conc {.}_{A}}\right)$ wherein:
Dose_{R} represents the dose (of (S)-3-(2,3-difluorophenyl)-3-methoxypyrrolidine, or a pharmaceutically acceptable salt thereof,) required to result in a plasma concentration within the reference range;
Dose_{A} represents the dose administered in step (i) and/or step (iv) (e.g. step (i)); Plasma conc._{R} represents a plasma concentration (e.g. Cmin or Ctrough (e.g. Ctrough)) within the reference range for (S)-3-(2,3-difluorophenyl)-3-methoxypyrrolidine; and Plasma conc._{A} represents the plasma concentration measured in step (ii) and/or step (v) (e.g. step (ii)).

If the concentration of a metabolite (e.g. 4-(2,3-difluorophenyl)-4-methoxy-2-pyrrolidinone ((S)-4-(2,3-difluorophenyl)-4-methoxy-2-pyrrolidinone) and/or 4-(2,3-difluorophenyl)-4-hydroxy-2-pyrrolidinone ((S)-4-(2,3-difluorophenyl)-4-hydroxy-2-pyrrolidinone) of the active ingredient is used to determine the dose of (S)-3-(2,3-difluorophenyl)-3-methoxypyrrolidine, or a pharmaceutically acceptable salt thereof, required to result in a plasma concentration within the reference range, this may be done by the same method as described above with respect to the active ingredient.

That is, the dose of (S)-3-(2,3-difluorophenyl)-3-methoxypyrrolidine, or a pharmaceutically acceptable salt thereof, required to result in a plasma concentration within the reference range for the metabolite(s) may be calculated from the (minimum) plasma concentration of 4-(2,3-difluorophenyl)-4-methoxy-2-pyrrolidinone ((S)-4-(2,3-difluorophenyl)-4-methoxy-2-pyrrolidinone) and/or 4-(2,3-difluorophenyl)-4-hydroxy-2-pyrrolidinone ((*S*)-4-(2,3-difluorophenyl)-4-hydroxy-2-pyrrolidinone measured in step (ii), and/or, optionally, step (v) (e.g. step (ii)), by determining the percentage increase in plasma concentration required to reach a plasma concentration within the reference range for 4-(2,3-difluorophenyl)-4-methoxy-2-pyrrolidinone ((S)-4-(2,3-difluorophenyl)-4-methoxy-2-pyrrolidinone), and increasing the dose administered by the same percentage.

Accordingly, the same equation as used above may also be used to calculate the required dose based on the plasma concentration of the metabolite. In which case, the Plasma conc._{A} and Plasma conc._{R} would refer to the plasma concentrations of the metabolite(s).

Alternatively, to account for variations in metabolism between subjects, the ratio of the steady state plasma concentrations (e.g. Cmin or Ctrough (for example Ctrough) of (S)-3-(2,3-difluorophenyl)-3-methoxypyrrolidine to its metabolite(s) (4-(2,3-difluorophenyl)-4-methoxy-2-pyrrolidinone ((S)-4-(2,3-difluorophenyl)-4-methoxy-2-pyrrolidinone) and/or 4-(2,3-difluorophenyl)-4-hydroxy-2-pyrrolidinone ((*S*)-4-(2,3-difluorophenyl)-4-hydroxy-2-pyrrolidinone) at steady state in an individual subject may first be determined and this may be used to calculate the dose of (S)-3-(2,3-difluorophenyl)-3-methoxypyrrolidine, or a pharmaceutically acceptable salt thereof, required to result in a plasma concentration within the reference range for the relevant metabolite.

It has surprisingly been found that 4-(2,3-difluorophenyl)-4-methoxy-2-pyrrolidinone (e.g. S-4-(2,3-difluorophenyl)-4-methoxy-2-pyrrolidinone) has a longer half-life than (S)-3-(2,3-difluorophenyl)-3-methoxypyrrolidine, and exhibits reduced intrapatient variation compared to active ingredient, such that measurement of the plasma concentration of this metabolite better reflects the overall exposure of the patient to the active ingredient. Thus, the data are less sensitive to the timing of sampling and analysis because of the less intra-individual variation. Therefore, when monitoring 4-(2,3-difluorophenyl)-4-methoxy-2-pyrrolidinone (e.g. S-4-(2,3-difluorophenyl)-4-methoxy-2-pyrrolidinone) rather than (S)-3-(2,3-difluorophenyl)-3-methoxypyrrolidine, the conclusions and predictions based thereupon are more reliable and in particular when the plasma collection is performed in an out-patient setting.

The metabolite 4-(2,3-difluorophenyl)-4-hydroxy-2-pyrrolidinone ((*S*)-4-(2,3-difluorophenyl)-4-hydroxy-2-pyrrolidinone) has also been found to have a considerably longer half-life than (S)-3-(2,3-difluorophenyl)-3-methoxypyrrolidine, and therefore may also better reflect the overall exposure to the active ingredient compared to the plasma concentration of the active ingredient, and may, therefore, also be beneficial to measure in a titration.

Accordingly, in particular embodiments of the titration, the minimum plasma concentration of 4-(2,3-difluorophenyl)-4-methoxy-2-pyrrolidinone (e.g. S-4-(2,3-difluorophenyl)-4-methoxy-2-pyrrolidinone) and/or 4-(2,3-difluorophenyl)-4-hydroxy-2-pyrrolidinone ((*S*)-4-(2,3-difluorophenyl)-4-hydroxy-2-pyrrolidinone) (particularly 4-(2,3-difluorophenyl)-4-methoxy-2-pyrrolidinone (e.g. S-4-(2,3-difluorophenyl)-4-methoxy-2-pyrrolidinone)) is used to determine the required dose of (S)-3-(2,3-difluorophenyl)-3-methoxypyrrolidine, or a pharmaceutically acceptable salt thereof.

In particular embodiments of this aspect, the ratio of the plasma concentrations of the active ingredient to its metabolite(s) is calculated, and used to calculate the dose required to provide a minimum plasma concentration within the reference ranges for (S)-3-(2,3-difluorophenyl)-3-methoxypyrrolidine and/or 4-(2,3-difluorophenyl)-4-methoxy-2-pyrrolidinone (e.g. (S)-3-(2,3-difluorophenyl)-3-methoxypyrrolidine) defined herein .

Accordingly, in particular embodiments, step (ii) comprises measuring the minimum plasma concentrations of (S)-3-(2,3-difluorophenyl)-3-methoxypyrrolidine and at least one of 4-(2,3-difluorophenyl)-4-methoxy-2-pyrrolidinone and 4-(2,3-difluorophenyl)-4-hydroxy-2-pyrrolidinone (e.g. 4-(2,3-difluorophenyl)-4-methoxy-2-pyrrolidinone).

In such embodiments, step (iii) particularly comprises, calculating the ratio between the minimum plasma concentration of (S)-3-(2,3-difluorophenyl)-3-methoxypyrrolidine and the minimum plasma concentration of 4-(2,3-difluorophenyl)-4-methoxy-2-pyrrolidinone and 4-(2,3-difluorophenyl)-4-hydroxy-2-pyrrolidinone (e.g. 4-(2,3-difluorophenyl)-4-methoxy-2-pyrrolidinone), and using this ratio to determine the dose of (S)-3-(2,3-difluorophenyl)-3-methoxypyrrolidine, or a pharmaceutically acceptable salt thereof, required to arrive at a plasma concentration within the reference ranges.

In alternative embodiments, the ratio between the plasma concentration of (S)-3-(2,3-difluorophenyl)-3-methoxypyrrolidine and at least one of 4-(2,3-difluorophenyl)-4-methoxy-2-pyrrolidinone and 4-(2,3-difluorophenyl)-4-hydroxy-2-pyrrolidinone (e.g. 4-(2,3-difluorophenyl)-4-methoxy-2-pyrrolidinone) is determined before commencing the titration.

In such embodiments, the titration may comprise the initial steps of:
(oi) administering an initial dose of (S)-3-(2,3-difluorophenyl)-3-methoxypyrrolidine, or a pharmaceutically acceptable salt thereof, (for example a dose of from about 25 to about 100 mg (of the active ingredient), e.g. about 50 mg, 1 to 3 times a day (e.g. 3 times)) to a subject during an initial time period sufficient to achieve a steady state plasma concentration of (S)-3-(2,3-difluorophenyl)-3-methoxypyrrolidine and at least one of 4-(2,3-difluorophenyl)-4-methoxy-2-pyrrolidinone ((S)-4-(2,3-difluorophenyl)-4-methoxy-2-pyrrolidinone) and 4-(2,3-difluorophenyl)-4-hydroxy-2-pyrrolidinone ((*S*)-4-(2,3-difluorophenyl)-4-hydroxy-2-pyrrolidinone) (in particular, 4-(2,3-difluorophenyl)-4-methoxy-2-pyrrolidinone ((S)-4-(2,3-difluorophenyl)-4-methoxy-2-pyrrolidinone), which time period may be from about 1 day to about 10 days (e.g from about 1 day to about 7 days);
(oii) measuring the minimum plasma concentration (Cmin or Ctrough (e.g. Ctrough)) of (S)-3-(2,3-difluorophenyl)-3-methoxypyrrolidine, and at least one of 4-(2,3-difluorophenyl)-4-methoxy-2-pyrrolidinone ((S)-4-(2,3-difluorophenyl)-4-methoxy-2-pyrrolidinone) and 4-(2,3-difluorophenyl)-4-hydroxy-2-pyrrolidinone ((*S*)-4-(2,3-difluorophenyl)-4-hydroxy-2-pyrrolidinone) (in particular, 4-(2,3-difluorophenyl)-4-methoxy-2-pyrrolidinone ((S)-4-(2,3-difluorophenyl)-4-methoxy-2-pyrrolidinone); and
(oiii) determining the ratio of the minimum plasma concentrations of (S)-3-(2,3-difluorophenyl)-3-methoxypyrrolidine to 4-(2,3-difluorophenyl)-4-methoxy-2-pyrrolidinone ((S)-4-(2,3-difluorophenyl)-4-methoxy-2-pyrrolidinone) and/or 4-(2,3-difluorophenyl)-4-hydroxy-2-pyrrolidinone ((*S*)-4-(2,3-difluorophenyl)-4-hydroxy-2-pyrrolidinone) (e.g. the ratio of (S)-3-(2,3-difluorophenyl)-3-methoxypyrrolidine to of 4-(2,3-difluorophenyl)-4-methoxy-2-pyrrolidinone ((S)-4-(2,3-difluorophenyl)-4-methoxy-2-pyrrolidinone) (based on the minimum plasma concentrations measured in step (oii).

In embodiments in which the ratio of the minimum plasma concentrations of (S)-3-(2,3-difluorophenyl)-3-methoxypyrrolidine to 4-(2,3-difluorophenyl)-4-methoxy-2-pyrrolidinone ((S)-4-(2,3-difluorophenyl)-4-methoxy-2-pyrrolidinone) and/or 4-(2,3-difluorophenyl)-4-hydroxy-2-pyrrolidinone ((*S*)-4-(2,3-difluorophenyl)-4-hydroxy-2-pyrrolidinone) is calculated before commencing the titration, step (ii) preferably comprises measuring the minimum plasma concentration of 4-(2,3-difluorophenyl)-4-methoxy-2-pyrrolidinone ((S)-4-(2,3-difluorophenyl)-4-methoxy-2-pyrrolidinone)) and/or 4-(2,3-difluorophenyl)-4-hydroxy-2-pyrrolidinone ((*S*)-4-(2,3-difluorophenyl)-4-hydroxy-2-pyrrolidinone) (e.g. of 4-(2,3-difluorophenyl)-4-methoxy-2-pyrrolidinone).

In such embodiments, step (iii) particularly comprises calculating the dose required to provide a minimum plasma concentration within the reference ranges for (S)-3-(2,3-difluorophenyl)-3-methoxypyrrolidine and/or 4-(2,3-difluorophenyl)-4-methoxy-2-pyrrolidinone (e.g. (S)-3-(2,3-difluorophenyl)-3-methoxypyrrolidine) defined herein from the minimum plasma concentration of 4-(2,3-difluorophenyl)-4-methoxy-2-pyrrolidinone and/or 4-(2,3-difluorophenyl)-4-hydroxy-2-pyrrolidinone (e.g. of 4-(2,3-difluorophenyl)-4-methoxy-2-pyrrolidinone) measured in step (ii) and, optionally, step (v), and the ratio of the minimum plasma concentrations of (S)-3-(2,3-difluorophenyl)-3-methoxypyrrolidine to 4-(2,3-difluorophenyl)-4-methoxy-2-pyrrolidinone ((S)-4-(2,3-difluorophenyl)-4-methoxy-2-pyrrolidinone) and/or 4-(2,3-difluorophenyl)-4-hydroxy-2-pyrrolidinone ((*S*)-4-(2,3-difluorophenyl)-4-hydroxy-2-pyrrolidinone) (e.g. 4-(2,3-difluorophenyl)-4-methoxy-2-pyrrolidinone ((S)-4-(2,3-difluorophenyl)-4-methoxy-2-pyrrolidinone).

In such embodiments, the minimum plasma concentration of (S)-3-(2,3-difluorophenyl)-3-methoxypyrrolidine for a specific dose (e.g. as administered in step (i) and/or step (iv)) may be calculated from the ratio of the (S)-3-(2,3-difluorophenyl)-3-methoxypyrrolidine to one of its metabolites (e.g. 4-(2,3-difluorophenyl)-4-methoxy-2-pyrrolidinone ((S)-4-(2,3-difluorophenyl)-4-methoxy-2-pyrrolidinone)) and the minimum plasma concentration of the metabolite measured in step (ii) and/or (v), for example, in accordance with the following equation: $Plasma {conc}_{AC} = \left(\frac{Plasma conc {.}_{API}}{Plasma conc {.}_{M}}\right) \times Plasma {conc}_{MA}$ wherein:
Plasma conc._{AC} represents the calculated minimum plasma concentration (e.g. Cmin or Ctrough (e.g. Ctrough)) of (S)-3-(2,3-difluorophenyl)-3-methoxypyrrolidine for a given dose (e.g. as administered in step (i) and/or (iv)).
Plasma conc._{API} represents the steady state minimum plasma concentration of (S)-3-(2,3-difluorophenyl)-3-methoxypyrrolidine at a given dose (e.g. as measured in step oii);
Plasma conc._{M} represents the steady state minimum plasma concentration of a metabolite of (S)-3-(2,3-difluorophenyl)-3-methoxypyrrolidine (e.g. 4-(2,3-difluorophenyl)-4-methoxy-2-pyrrolidinone ((S)-4-(2,3-difluorophenyl)-4-methoxy-2-pyrrolidinone)) at the same dose as the dose used for Plasma conc_{API} (e.g. as measured in step oii); and
Plasma conc._{MA} represents the minimum plasma concentration for the metabolite (e.g. 4-(2,3-difluorophenyl)-4-methoxy-2-pyrrolidinone ((S)-4-(2,3-difluorophenyl)-4-methoxy-2-pyrrolidinone)) measured for a given dose (e.g. as measured in step (ii) and/or step (v) (e.g. step (ii)).

The required dose can then be calculated from the calculated concentration of minimum plasma concentration of (S)-3-(2,3-difluorophenyl)-3-methoxypyrrolidine in accordance with the following equation. ${Dose}_{R} = {Dose}_{A} \times \left(\frac{Plasma conc {.}_{R}}{Plasma conc {.}_{AC}}\right)$ wherein:
Dose_{R} represents the dose (of (S)-3-(2,3-difluorophenyl)-3-methoxypyrrolidine, or a pharmaceutically acceptable salt thereof,) required to result in a plasma concentration within the reference range;
Dose_{A} represents the dose administered in step (i) and/or step (iv) (e.g. step (i)); Plasma conc._{R} represents a plasma concentration (e.g. Cmin or Ctrough (e.g. Ctrough)) within the reference range for (S)-3-(2,3-difluorophenyl)-3-methoxypyrrolidine; and Plasma conc._{AC} represents the calculated plasma concentration of (S)-3-(2,3-difluorophenyl)-3-methoxypyrrolidine, determined using the method described herein, based on the minimum plasma concentration of the relevant metabolite measured in step (ii) and/or step (v) (e.g. step (ii)), following the administration of DoseA in step(i) and/or step (iv) (e.g. step (i)).

An advantage of this approach is that, once the steady state minimum plasma concentration ratio of active ingredient to metabolite (e.g. 4-(2,3-difluorophenyl)-4-methoxy-2-pyrrolidinone (e.g. S-4-(2,3-difluorophenyl)-4-methoxy-2-pyrrolidinone) is known, the minimum plasma concentration of the metabolite can be used for the ongoing titration (e.g. in steps (ii) and/or step (v)), and so provide more reliable data regarding the required concentration of the active ingredient, particularly if the plasma collection for the titration is performed in an out-patient setting.

The uses and methods described herein provide new treatments for disorders associated with neurodegenerative diseases. In particular, the disorder associated with a neurodegenerative disease is a cognitive disorder or a movement disorder. More particularly, the disorder associated with a neurodegenerative disease is a movement disorder, such as impaired balance or postural instability.

The skilled person will understand that references to the treatment of disorders associated with neurodegenerative diseases will take its normal meaning in the field of medicine. In particular, the treatment may refer to achieving a reduction in the severity and/or frequency of occurrence of one or more clinical symptom associated with the condition, as adjudged by a physician attending a patient having or being susceptible to such symptoms.

Particular neurodegenerative diseases that may be mentioned include Alzheimer's disease and Parkinson's disease and other dementia disorders (such as dementia with Lewy bodies). More particularly, the neurodegenerative disease is Alzheimer's disease or Parkinson's disease.

In particular embodiments, the neurodegenerative disease is Parkinson's disease.

In particular embodiments, the disorder associated with a neurodegenerative disease is a cognitive disorder or a movement disorder.

Cognitive disorders that may be mentioned include mild cognitive impairment, cognitive dysfunction and memory loss.

Movement disorders that may be mentioned include postural instability.

In further particular embodiments, the treatment of the neurodegenerative disease or disorder (e.g. Parkinson's disease) comprises the treatment of postural instability.

Postural instability is a well-known and common feature of Parkinson's disease, which includes poor balance, unsteady gait, difficultly standing or walking and frequent falls. Patients may also experience dizziness, vertigo and/or disorientation. By treatment of postural instability, we include achieving a reduction in the severity and/or frequency of any of these symptoms. In particular, we include reducing the frequency of falls experienced by the subject. We also include preventative or prophylactic treatment of postural stability, which may refer to achieving a reduction of (for example, at least a 10% reduction, such as at least a 20%, 30% or 40% reduction, e.g. at least a 50% reduction) in the likelihood of the subject experiencing postural instability and/or any of the associated symptoms. In particular, we include preventing the subject from experiencing falls as well as reducing the frequency of falls.

As used herein, references to a subject will refer to a living subject in need of treatment, including mammalian (e.g. human) subjects. In particular, references to a subject will refer to human subjects. The term subject may also be replaced by patient and vice versa.

For the avoidance of doubt, the skilled person will understand that such treatment (including preventative or prophylactic treatment) will be performed in a patient (or subject) in need thereof. The need of a patient (or subject) may be assessed by those skilled the art using routine techniques.

As used herein, the term effective amount will refer to an amount of a compound that confers a therapeutic effect on the treated patient. The effect may be observed in a manner that is objective (i.e. measurable by some test or marker) or subjective (i.e. the subject gives an indication of and/or feels an effect). In particular, the effect may be observed (e.g. measured) in a manner that is objective, using appropriate tests as known to those skilled in the art.

More particularly, effective amount may be understood to refer to the amount necessary to provide a beneficial effect in the subject being treated. As the effectiveness of (S)-3-(2,3-difluorophenyl)-3-methoxypyrrolidine in the treatment of neurodegenerative diseases and disorders associated therewith (as described herein), such as Parkinson's disease, and particularly the postural instability associated therewith, is dependent on the level of exposure of the active ingredient in blood plasma, the absolute dosage required to be administered may vary between patients. However, an effective dose of (S)-3-(2,3-difluorophenyl)-3-methoxypyrrolidine may be understood as being an amount sufficient to provide a plasma concentration that achieves a beneficial effect (including, particularly, a reduction in postural instability and/or associated falls).

In the methods and uses described herein, the compound (S)-3-(2,3-difluorophenyl)-3-methoxypyrrolidine, or a pharmaceutically acceptable salt thereof, is administered in an amount that results in a minimum plasma concentration (e.g. Cmin or Ctrough) of (S)-3-(2,3-difluorophenyl)-3-methoxypyrrolidine of from about 500 nM to about 3000 nM during a dosing interval (for example before dosing in the morning); and/or (ii) a minimum plasma concentration (e.g Cmin or Ctrough) of 4-(2,3-difluorophenyl)-4-methoxy-2-pyrrolidinone of from about 800 nM to about 3200 nM during a dosing interval (for example before dosing in the morning).

In particular embodiments, the compound (S)-3-(2,3-difluorophenyl)-3-methoxypyrrolidine, or a pharmaceutically acceptable salt thereof, is administered in an amount that results in a minimum plasma concentration (e.g. Cmin or Ctrough) of (S)-3-(2,3-difluorophenyl)-3-methoxypyrrolidine of from about 500 nM to about 2500 nM during a dosing interval (for example before dosing in the morning). More particularly, the minimum plasma concentration (e.g. Cmin or Ctrough) of (S)-3-(2,3-difluorophenyl)-3-methoxypyrrolidine during a dosing interval is from about 500 nM to about 2000 nM, such as from about 500 nM to about 1500 nM, most particularly from about 500 nM to about 1000 nM (for example before dosing in the morning).

In particular embodiments, the compound (S)-3-(2,3-difluorophenyl)-3-methoxypyrrolidine, or a pharmaceutically acceptable salt thereof, is administered in an amount that results in a minimum plasma concentration (e.g. Cmin or Ctrough) of 4-(2,3-difluorophenyl)-4-methoxy-2-pyrrolidinone of from about 800 nM to about 2400 nM during a dosing interval. More particularly, the minimum plasma concentration of 4-(2,3-difluorophenyl)-4-methoxy-2-pyrrolidinone during a dosing interval is from about 800 nM to about 1600 nM.

4-(2,3-difluorophenyl)-4-methoxy-2-pyrrolidinone is a metabolite of (S)-3-(2,3-difluorophenyl)-3-methoxypyrrolidine. In the methods described herein, the plasma concentration of 4-(2,3-difluorophenyl)-4-methoxy-2-pyrrolidinone was determined using achiral chromatography, and therefore the enantiomeric purity of the metabolite was not assessed. However, it is believed that the compound is present as the single (S)-enantiomer, (S)-4-(2,3-difluorophenyl)-4-methoxy-2-pyrrolidinone as the metabolite is not expected to racemise, or partly racemise, in vivo. Accordingly, references herein to 4-(2,3-difluorophenyl)-4-methoxy-2-pyrrolidinone and the plasma concentration of this compound may particularly be understood to refer to (S)-4-(2,3-difluorophenyl)-4-methoxy-2-pyrrolidinone. Thus, in particular embodiments, the 4-(2,3-difluorophenyl)-4-methoxy-2-pyrrolidinone is (S)-4-(2,3-difluorophenyl)-4-methoxy-2-pyrrolidinone.

When used herein in relation to a specific value (such as an amount), the term "about" (or similar terms, such as "approximately") will be understood as indicating that such values may vary by up to 10% (particularly, up to 5%, such as up to 1%) of the value defined. It is contemplated that, at each instance, such terms may be replaced with the notation "±10%", or the like (or by indicating a variance of a specific amount calculated based on the relevant value). It is also contemplated that, at each instance, such terms may be deleted.

The methods and uses described herein involve achieving the minimum plasma concentration of (S)-3-(2,3-difluorophenyl)-3-methoxypyrrolidine and/or its metabolite 4-(2,3-difluorophenyl)-4-methoxy-2-pyrrolidinone (e.g. (S)-3-(2,3-difluorophenyl)-3-methoxypyrrolidine). In certain embodiments, the plasma concentration of both (S)-3-(2,3-difluorophenyl)-3-methoxypyrrolidine and/or its metabolite 4-(2,3-difluorophenyl)-4-methoxy-2-pyrrolidinone (e.g. (S)-3-(2,3-difluorophenyl)-3-methoxypyrrolidine) are measured. In other embodiments, the plasma concentration of only one of the compounds is measured.

The methods and uses described herein involve administering (S)-3-(2,3-difluorophenyl)-3-methoxypyrrolidine, or a pharmaceutically acceptable salt thereof, in an amount that results in the required minimum plasma concentration of (S)-3-(2,3-difluorophenyl)-3-methoxypyrrolidine and/or its metabolite 4-(2,3-difluorophenyl)-4-methoxy-2-pyrrolidinone (e.g. (S)-3-(2,3-difluorophenyl)-3-methoxypyrrolidine) is achieved during a dosing interval (i.e the time between two doses). As used herein, the minimum plasma concentration of a compound during a dosing interval may be understood to include Cmin (the minimum steady state value for plasma concentration between a dosing interval) and Ctrough (the plasma concentration of a compound immediately before the next dose is administered). In particular embodiments, the minimum plasma concentration during a dosing interval is Cmin. In alternative embodiments, the minimum plasma concentration during a dosing interval is Ctrough. It is also included that Cmin and Ctrough may be the same value.

In this document, unless otherwise stated, the plasma concentration, such as the minimum plasma concentration (e.g. Cmin or Ctrough, particularly, Ctrough), may refer to the plasma concentration, during the treatment period, in the morning before administration of the dose (e.g. the first dose, or only dose, of the day).

In particular embodiments of the medical uses and methods described herein, the plasma concentration of (S)-3-(2,3-difluorophenyl)-3-methoxypyrrolidine and 4-(2,3-difluorophenyl)-4-methoxy-2-pyrrolidinone (including (S)-4-(2,3-difluorophenyl)-4-methoxy-2-pyrrolidinone) is measured by liquid chromatography (and falls within the stated ranges when measured by this method). More particularly, the plasma concentration is measured by liquid chromatography-mass spec or liquid chromatography with tandem mass spectrometry (LC-MS/MS), such as UPLC-MS/MS. Most particularly, the plasma concentration is measured by liquid chromatography with tandem mass spectrometry (LC-MS/MS), such as UPLC-MS/MS.

Appropriate methods for measuring analyte concentration by liquid chromatography (including liquid chromatography-mass spectrometry (LC-MS) and liquid chromatography with tandem mass spectrometry (LC-MS/MS)) are known to the skilled person. Appropriate methods may also be determined by analogy to the methods described herein. In particular, the method may be calibrated using internal standards of the compounds of interest of (S)-3-(2,3-difluorophenyl)-3-methoxypyrrolidine and 4-(2,3-difluorophenyl)-4-methoxy-2-pyrrolidinone (including (S)-4-(2,3-difluorophenyl)-4-methoxy-2-pyrrolidinone).

As described above, determining an appropriate dosage to achieve the effective plasma concentrations described herein may also be achieved routinely by the skilled person. For example, the appropriate dosage may be determined by titration prior to commencing treatment and/or during treatment, as appropriate.

For example, in a drug titration, a subject may first be treated with a low dose of (S)-3-(2,3-difluorophenyl)-3-methoxypyrrolidine, or a pharmaceutically acceptable salt thereof (e.g. 50 mg of the active ingredient 1 to 3 times a day) and the minimum plasma concentration (e.g. Cmin or Ctrough) of the (S)-3-(2,3-difluorophenyl)-3-methoxypyrrolidine, 4-(2,3-difluorophenyl)-4-methoxy-2-pyrrolidinone (including (S)-4-(2,3-difluorophenyl)-4-methoxy-2-pyrrolidinone) during a dosing interval may be measured such as measured at steady state then the dose progressively increased (e.g. by 50 mg a day) until the minimum plasma concentrations of (S)-3-(2,3-difluorophenyl)-3-methoxypyrrolidine and/or 4-(2,3-difluorophenyl)-4-methoxy-2-pyrrolidinone (including (S)-4-(2,3-difluorophenyl)-4-methoxy-2-pyrrolidinone) during a dosing interval are within the effectives range as defined herein.

Accordingly, in particular embodiments, the treatment comprises determining the dose of (S)-3-(2,3-difluorophenyl)-3-methoxypyrrolidine required to result in the plasma concentration of (S)-3-(2,3-difluorophenyl)-3-methoxypyrrolidine and/or 4-(2,3-difluorophenyl)-4-methoxy-2-pyrrolidinone as defined in any one of Claims 1 to 15, comprising the steps of:
(a) administering (S)-3-(2,3-difluorophenyl)-3-methoxypyrrolidine, or a pharmaceutically acceptable salt thereof, to the subject;
(b) measuring the plasma concentration of (S)-3-(2,3-difluorophenyl)-3-methoxypyrrolidine and/or 4-(2,3-difluorophenyl)-4-methoxy-2-pyrrolidinone in the subject;
(c) comparing the plasma concentrations of the (S)-3-(2,3-difluorophenyl)-3-methoxypyrrolidine and/or 4-(2,3-difluorophenyl)-4-methoxy-2-pyrrolidinone to the concentrations defined in Claim 1 or Claim 12;
(d) determining whether the plasma concentration of (S)-3-(2,3-difluorophenyl)-3-methoxypyrrolidine and/or 4-(2,3-difluorophenyl)-4-methoxy-2-pyrrolidinone (e.g. (S)-4-(2,3-difluorophenyl)-4-methoxy-2-pyrrolidinone) is within the reference range for the treatment of a disease associated with a neurodegenerative disease and related disorders; and, if not, adjusting the dose of (S)-3-(2,3-difluorophenyl)-3-methoxypyrrolidine administered to the subject to provide a dose that results in a plasma concentration within the effective range for the subject being treated treated (and then proceeding with the treatment using the dose of (S)-3-(2,3-difluorophenyl)-3-methoxypyrrolidine required to result in a plasma concentration within the effective range for the patient being treated).

In a further aspect of the invention, there is provided the compound (S)-3-(2,3-difluorophenyl)-3-methoxypyrrolidine, or a pharmaceutically acceptable salt thereof, for use in the treatment of a disorder associated with a neurodegenerative disease (as defined herein), wherein the treatment comprises the steps of:
(a) administering (S)-3-(2,3-difluorophenyl)-3-methoxypyrrolidine, or a pharmaceutically acceptable salt thereof, to a subject;
(b) measuring the plasma concentration of (S)-3-(2,3-difluorophenyl)-3-methoxypyrrolidine and/or 4-(2,3-difluorophenyl)-4-methoxy-2-pyrrolidinone (e.g. (S)-4-(2,3-difluorophenyl)-4-methoxy-2-pyrrolidinone);
(c) comparing the plasma concentrations of the (S)-3-(2,3-difluorophenyl)-3-methoxypyrrolidine and/or 4-(2,3-difluorophenyl)-4-methoxy-2-pyrrolidinone (e.g. (S)-4-(2,3-difluorophenyl)-4-methoxy-2-pyrrolidinone) to a reference range for effective treatment; and
(d) determining whether the plasma concentration of (S)-3-(2,3-difluorophenyl)-3-methoxypyrrolidine and/or 4-(2,3-difluorophenyl)-4-methoxy-2-pyrrolidinone (e.g. (S)-4-(2,3-difluorophenyl)-4-methoxy-2-pyrrolidinone) is within the reference range for the treatment of a disease associated with a neurodegenerative disease and related disorders; and, if not, adjusting the dose of (S)-3-(2,3-difluorophenyl)-3-methoxypyrrolidine administered to the subject to provide a dose that results in a plasma concentration within the effective range for the subject being treated (and then proceeding with the treatment using the dose of (S)-3-(2,3-difluorophenyl)-3-methoxypyrrolidine required to result in a plasma concentration within the effective range for the patient being treated).

In particular embodiments of this aspect of the invention, the reference ranges for the plasma concentrations of (S)-3-(2,3-difluorophenyl)-3-methoxypyrrolidine and/or 4-(2,3-difluorophenyl)-4-methoxy-2-pyrrolidinone (e.g. (S)-4-(2,3-difluorophenyl)-4-methoxy-2-pyrrolidinone) are as defined herein, i.e.
(i) a minimum plasma concentration (e.g. Cmin or Ctrough) of (S)-3-(2,3-difluorophenyl)-3-methoxypyrrolidine of from about 500 nM to about 3000 nM (such as from about 500 nM to about 2500 nM, for example from about 500 nM to about 2000 nM, or from about 500 nM to about 1500 nM, e.g. from about 500 nM to about 1000 nM) during a dosing interval (for example before dosing in the morning); and/or
(ii) a minimum plasma concentration (e.g. Cmin or Ctrough) of 4-(2,3-difluorophenyl)-4-methoxy-2-pyrrolidinone of about 800 nM to about 3200 nM (such as from about 800 nM to about 2400 nm, e.g. from about 800 nM to about 1600 nM) during a dosing interval (for example before dosing in the morning).

In particular embodiments of this aspect, step (a) involves administering a low dose of (S)-3-(2,3-difluorophenyl)-3-methoxypyrrolidine, or a pharmaceutically acceptable salt thereof, (for example a dose of 25-50-100 mg (e.g. 50 mg) 1 to 3 times a day for a period of from 1 to 10 days ) to a subject; and step (d) involves, optionally repeatedly, increasing the dose (for example by 50 to 100 mg per dosage period (which may for, example be, 1 to 10 days, 1 to 7 days or 1 day)) in order to determine an effective dose for the subject being treated.

### Metabolites and methods of determining an effective dose of (S)-3-(2,3-difluorophenyl)-3-methoxypyrrolidine

As described herein, the compound 4-(2,3-difluorophenyl)-4-methoxy-2-pyrrolidinone (particularly (*S*)-4-(2,3-difluorophenyl)-4-methoxy-2-pyrrolidinone) is a metabolite of (S)-3-(2,3-difluorophenyl)-3-methoxypyrrolidine.

A further metabolite of the compound is 4-(2,3-difluorophenyl)-4-hydroxy-2-pyrrolidinone, and particularly (*S*)-4-(2,3-difluorophenyl)-4-hydroxy-2-pyrrolidinone.

These compounds and deuterated variants thereof can be used to analyse the pharmacokinetics of (S)-3-(2,3-difluorophenyl)-3-methoxypyrrolidine, for example to monitor the level of exposure to the active ingredient, and therefore to determine the optimum dose for the subject being treated.

Accordingly, in a further aspect of the invention, there is provided a compound, a pharmaceutically acceptable salt or deuterated variant thereof, selected from the group consisting of:
4-(2,3-difluorophenyl)-4-methoxy-2-pyrrolidinone 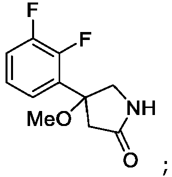
(*S*)-4-(2,3-difluorophenyl)-4-methoxy-2-pyrrolidinone 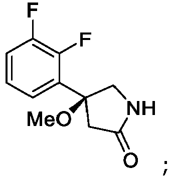
4-(2,3-difluorophenyl)-4-hydroxy-2-pyrrolidinone 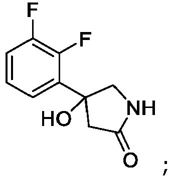 and
(*S*)-4-(2,3-difluorophenyl)-4-hydroxy-2-pyrrolidinone

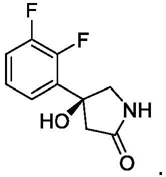

Particular deuterated variants that may be mentioned include 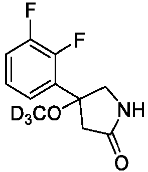 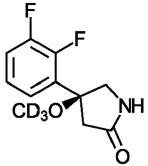 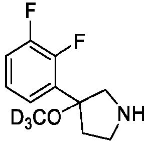 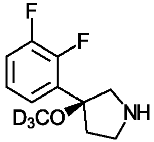 and, particularly the (S)-enantiomer of the compounds.

In a further aspect of the invention, there is provided the compound (S)-3-(2,3-difluorophenyl)-3-methoxypyrrolidine, or a pharmaceutically acceptable salt thereof, for use in the treatment of a disorder associated with a neurodegenerative disease (as defined herein), wherein the treatment comprises the steps of:
(a) administering (S)-3-(2,3-difluorophenyl)-3-methoxypyrrolidine, or a pharmaceutically acceptable salt thereof, to a subject;
(b) measuring the plasma concentration of one or more metabolite of (S)-3-(2,3-difluorophenyl)-3-methoxypyrrolidine selected from the group consisting of
   4-(2,3-difluorophenyl)-4-methoxy-2-pyrrolidinone;
   (*S*)-4-(2,3-difluorophenyl)-4-methoxy-2-pyrrolidinone;
   4-(2,3-difluorophenyl)-4-hydroxy-2-pyrrolidinone; and
   (*S*)-4-(2,3-difluorophenyl)-4-hydroxy-2-pyrrolidinone
(c) comparing the plasma concentrations of one or more of
   4-(2,3-difluorophenyl)-4-methoxy-2-pyrrolidinone;
   (*S*)-4-(2,3-difluorophenyl)-4-methoxy-2-pyrrolidinone;
   4-(2,3-difluorophenyl)-4-hydroxy-2-pyrrolidinone; and
   (*S*)-4-(2,3-difluorophenyl)-4-hydroxy-2-pyrrolidinone
   to a reference range for effective treatment; and
(d) determining whether the plasma concentration of the one or more of
   4-(2,3-difluorophenyl)-4-methoxy-2-pyrrolidinone;
   (*S*)-4-(2,3-difluorophenyl)-4-methoxy-2-pyrrolidinone;
   4-(2,3-difluorophenyl)-4-hydroxy-2-pyrrolidinone; and
   (*S*)-4-(2,3-difluorophenyl)-4-hydroxy-2-pyrrolidinone
   that was measured is within the reference range for the treatment of a disease associated with a neurodegenerative disease and related disorders; and, if not, adjusting the dose of (S)-3-(2,3-difluorophenyl)-3-methoxypyrrolidine administered to the subject to provide a dose that results in a plasma concentration corresponding to the effective range for the subject being treated (and then proceeding with the treatment using the dose of (S)-3-(2,3-difluorophenyl)-3-methoxypyrrolidine so determined)..

In particular embodiments of this aspect, the reference range for 4-(2,3-difluorophenyl)-4-methoxy-2-pyrrolidinone (or (*S*)-4-(2,3-difluorophenyl)-4-methoxy-2-pyrrolidinone) is as defined herein above with respect to the other aspects of the invention.

In a further aspect of the invention, there is provided a method of determining an effective dose of (S)-3-(2,3-difluorophenyl)-3-methoxypyrrolidine, or a pharmaceutically acceptable salt thereof, for the treatment of a disorder associated with a neurodegenerative disease, comprising the steps of:
(a) administering the compound ((S)-3-(2,3-difluorophenyl)-3-methoxypyrrolidine, or a pharmaceutically acceptable salt thereof, to a subject;
(b) obtaining a plasma sample from the subject;
(c) measuring the concentration of one or more metabolites of (S)-3-(2,3-difluorophenyl)-3-methoxypyrrolidine, selected from the group consisting of:
   4-(2,3-difluorophenyl)-4-methoxy-2-pyrrolidinone;
   (*S*)-4-(2,3-difluorophenyl)-4-methoxy-2-pyrrolidinone;
   4-(2,3-difluorophenyl)-4-hydroxy-2-pyrrolidinone; and
   (*S*)-4-(2,3-difluorophenyl)-4-hydroxy-2-pyrrolidinone in the plasma sample;
(d) comparing the plasma concentration of the one or more metabolites to a reference value (e.g. a range); and
(e) determining an effective dose of (S)-3-(2,3-difluorophenyl)-3-methoxypyrrolidine for the treatment of a disorder associated with a neurodegenerative disease.

In particular embodiments of this aspect of the invention, the metabolite of (S)-3-(2,3-difluorophenyl)-3-methoxypyrrolidine that the plasma concentration is measured for in step (c) is 4-(2,3-difluorophenyl)-4-methoxy-2-pyrrolidinone or, particularly, (*S*)-4-(2,3-difluorophenyl)-4-methoxy-2-pyrrolidinone. In such embodiments, the reference value for the plasma concentration described in step (c) minimum plasma concentration (e.g. Cmin or Ctrough) of 4-(2,3-difluorophenyl)-4-methoxy-2-pyrrolidinone of about 800 nM to about 3200 nM (such as from about 800 nM to about 2400 nm, e.g. from about 800 nM to about 1600 nM) during a dosing interval (for example before dosing in the morning).

In particular embodiments of this aspect, step (a) involves administering a low dose of (S)-3-(2,3-difluorophenyl)-3-methoxypyrrolidine, or a pharmaceutically acceptable salt thereof, (for example a dose of 50 mg 1 to 3 times a day) to a subject; and step (e) involves, optionally repeatedly, increasing the dose (for example by 50 to 100 mg a day) in order to determine an effective dose for the subject being treated.

In particular embodiments, step (e) comprises determining whether the plasma concentration of the metabolite that was measured is within the reference value (range)_ for the treatment of a disease associated with a neurodegenerative disease and related disorders; and, if not, adjusting the dose of (S)-3-(2,3-difluorophenyl)-3-methoxypyrrolidine administered to the subject to provide a dose that results in a plasma concentration within the effective range for the subject being treated.

In particular embodiments of this aspect, step (a) involves administering a low dose of (S)-3-(2,3-difluorophenyl)-3-methoxypyrrolidine, or a pharmaceutically acceptable salt thereof, (for example a dose of 50 mg 1 to 3 times a day) to a subject; and step (d) involves, optionally repeatedly, increasing the dose (for example by 50 to 100 mg a day) in order to determine an effective dose for the subject being treated.

### Dosage forms

The compound (S)-3-(2,3-difluorophenyl)-3-methoxypyrrolidine, or a pharmaceutically acceptable salt thereof, may be administered in the form of a pharmaceutical composition, or formulation, comprising the active ingredient and, optionally, one or more pharmaceutically acceptable excipients.

As used herein, the term pharmaceutically-acceptable excipients includes references to vehicles, adjuvants, carriers, diluents, pH adjusting and buffering agents, tonicity adjusting agents, stabilizers, wetting agents and the like. In particular, such excipients may include adjuvants, diluents or carriers.

In particular embodiments, the pharmaceutical composition comprises at least one pharmaceutically-acceptable excipient.

The skilled person will understand that the compound (S)-3-(2,3-difluorophenyl)-3-methoxypyrrolidine, or a pharmaceutically acceptable salt thereof, will normally be administered by an appropriate route to act systemically. For example, the compound, or pharmaceutically acceptable salt thereof may be administered orally, intravenously, subcutaneously, buccally, rectally, dermally, nasally, tracheally, bronchially, sublingually, intranasally, topically, by any other parenteral route or *via* inhalation, in a pharmaceutically acceptable dosage form. In particular, compound (S)-3-(2,3-difluorophenyl)-3-methoxypyrrolidine, or a pharmaceutically acceptable salt thereof, may be administered orally.

Pharmaceutical compositions as described herein will include compositions in the form of tablets, capsules or elixirs for oral administration, suppositories for rectal administration, sterile solutions or suspensions for parenteral or intramuscular administration, and the like.

Thus, in particular embodiments, the pharmaceutical formulation is provided in a pharmaceutically acceptable dosage form, including tablets or capsules, liquid forms to be taken orally or by injection, suppositories, creams, gels, foams, inhalants (e.g. to be applied intranasally), or forms suitable for topical administration. For the avoidance of doubt, in such embodiments, compounds of the invention may be present as a solid (e.g. a solid dispersion), liquid (e.g. in solution) or in other forms, such as in the form of micelles.

For example, in the preparation of pharmaceutical formulations for oral administration, the compound may be mixed with solid, powdered ingredients such as lactose, saccharose, sorbitol, mannitol, starch, amylopectin, cellulose derivatives, gelatin, or another suitable ingredient, as well as with disintegrating agents and lubricating agents such as magnesium stearate, calcium stearate, sodium stearyl fumarate and polyethylene glycol waxes. The mixture may then be processed into granules or compressed into tablets.

Soft gelatin capsules may be prepared with capsules containing one or more active compounds (e.g. compounds of the first and, therefore, second and third aspects of the invention, and optionally additional therapeutic agents), together with, for example, vegetable oil, fat, or other suitable vehicle for soft gelatin capsules. Similarly, hard gelatine capsules may contain such compound(s) in combination with solid powdered ingredients such as lactose, saccharose, sorbitol, mannitol, potato starch, corn starch, amylopectin, cellulose derivatives or gelatin.

Dosage units for rectal administration may be prepared (i) in the form of suppositories which contain the compound(s) mixed with a neutral fat base; (ii) in the form of a gelatin rectal capsule which contains the active substance in a mixture with a vegetable oil, paraffin oil, or other suitable vehicle for gelatin rectal capsules; (iii) in the form of a ready-made micro enema; or (iv) in the form of a dry micro enema formulation to be reconstituted in a suitable solvent just prior to administration.

Liquid preparations for oral administration may be prepared in the form of syrups or suspensions, e.g. solutions or suspensions, containing the compound(s) and the remainder of the formulation consisting of sugar or sugar alcohols, and a mixture of ethanol, water, glycerol, propylene glycol and polyethylene glycol. If desired, such liquid preparations may contain colouring agents, flavouring agents, saccharine and carboxymethyl cellulose or other thickening agent. Liquid preparations for oral administration may also be prepared in the form of a dry powder to be reconstituted with a suitable solvent prior to use.

Solutions for parenteral administration may be prepared as a solution of the compound(s) in a pharmaceutically acceptable solvent. These solutions may also contain stabilizing ingredients and/or buffering ingredients and are dispensed into unit doses in the form of ampoules or vials. Solutions for parenteral administration may also be prepared as a dry preparation to be reconstituted with a suitable solvent extemporaneously before use.

Depending on e.g. potency and physical characteristics of the compound of the invention (i.e. active ingredient), pharmaceutical formulations that may be mentioned include those in which the active ingredient is present in an amount that is at least 1% (or at least 10%, at least 30% or at least 50%) by weight. That is, the ratio of active ingredient to the other components (i.e. the addition of adjuvant, diluent and carrier) of the pharmaceutical composition is at least 1:99 (or at least 10:90, at least 30:70 or at least 50:50) by weight.

The invention will be further described by reference to the following examples, which are not intended to limit the scope of the invention.

### BRIEF DESCRIPTION OF THE FIGURES

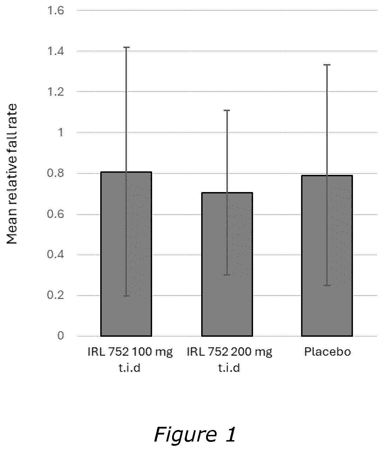

Figure 1 shows the mean relative fall rate observed for the different treatment groups (IRL752 lower dose (300 mg per day) IRL752 higher dose (600 mg per day), and placebo) in the clinical study. The relative fall rates in the treatment groups were not found to be significantly different to the placebo group.

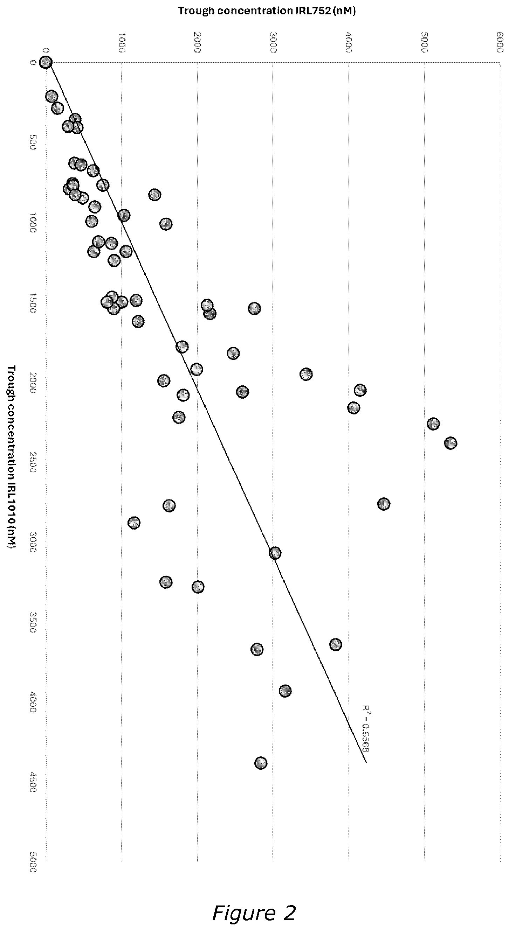

Figure 2 shows the trough plasma concentration of IRL752 (y axis) against the trough plasma concentration of IRL1010 (x axis) at week 6 of the clinical study. A clear correlation between the plasma concentration of IRL752 and IRL1010 can be seen.

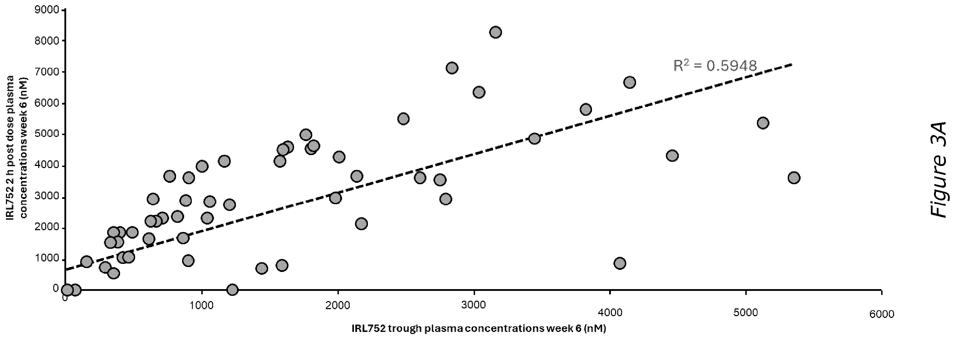

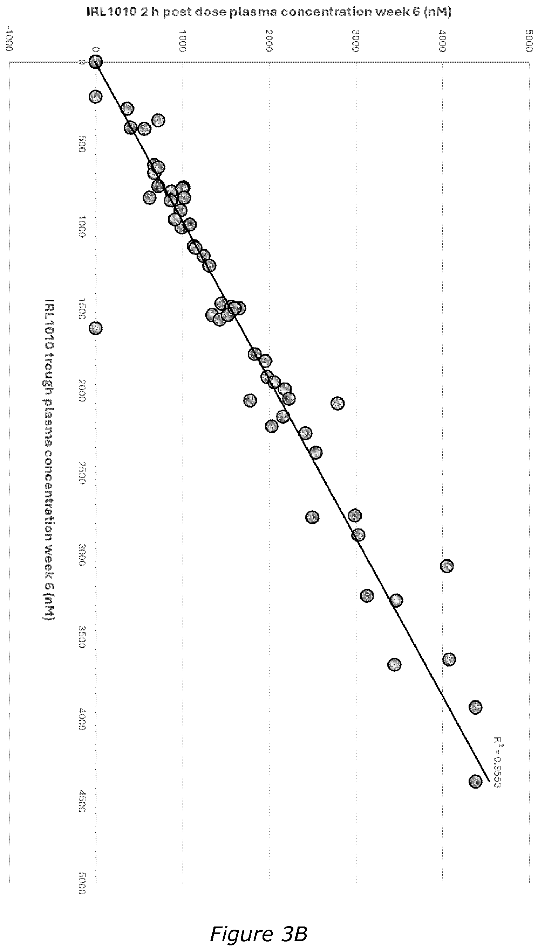

Figures 3A and 3B show the pre-dose plasma concentration (measured immediately before administration of the morning dose; x axis) of IRL752 (Figure 3A) and IRL1010 (Figure 3B) compared to the plasma concentration at two hours post dose (measured two hours after administration of the morning dose; y axis) plasma concentration, as measured at week 6. IRL1010 was found to have a longer half-life than IRL752, leading to more stable plasma concentration over 24 hours (and therefore less sensitive to the timing of the dose vs sampling). IRL1010 plasma concentrations also showed smaller intraindividual variability, more accurately reflecting the overall exposure to the active ingredient. Each dot represents a study participant.

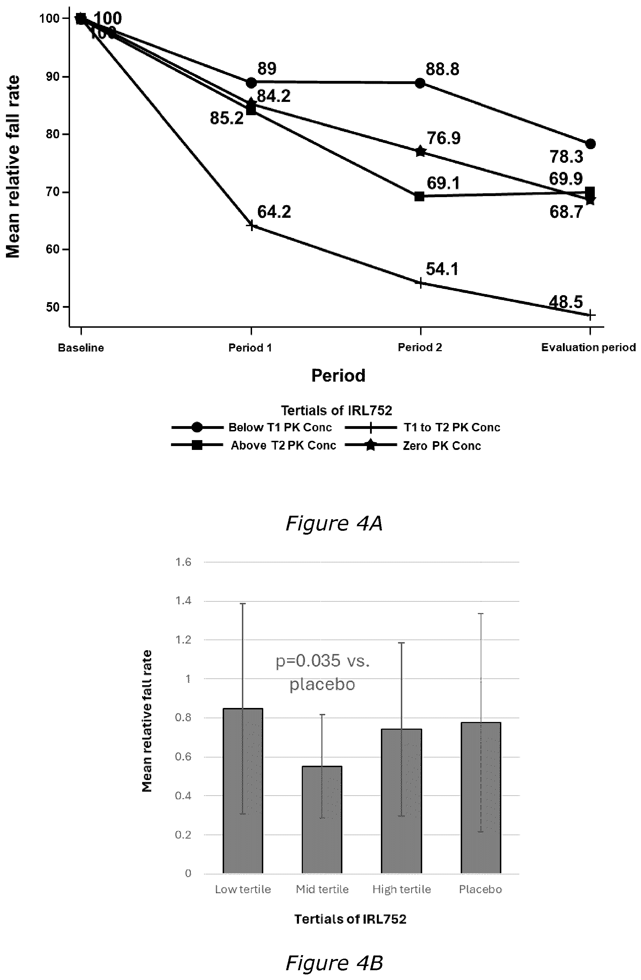

Figures 4A and 4B show the mean fall rates observed in the three tertials (Below T1, T1 to T2 and above T2) of IRL752 plasma concentration. A significant reduction in fall rate was observed in the T1 to T2 tertile over the whole treatment period (12 weeks). In Figure 4A, the baseline value of 100 on the y-axis refers to a reference value for the study participants prior to being subjected to the treatment. Further, "Period 1" stands for the first month of treatment, "Period 2" stands for the second month of treatment and "Evaluation period " stands for the last month of treatment.

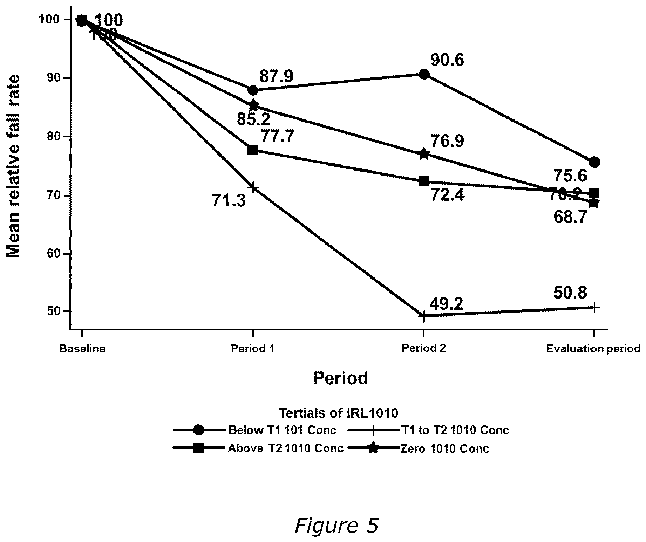

Figure 5 shows the mean fall rates observed in the three tertials (Below T1, T1 to T2 and above T2) of IRL1010 plasma concentration. A significant reduction in fall rate was observed in the T1 to T2 tertile over the whole treatment period. The baseline value of 100 on the y-axis refers to a reference value for the study participants prior to being subjected to the treatment. Further, "Period 1" stands for the first month of treatment, "Period 2" stands for the second month of treatment and "Evaluation period " stands for the last month of treatment.

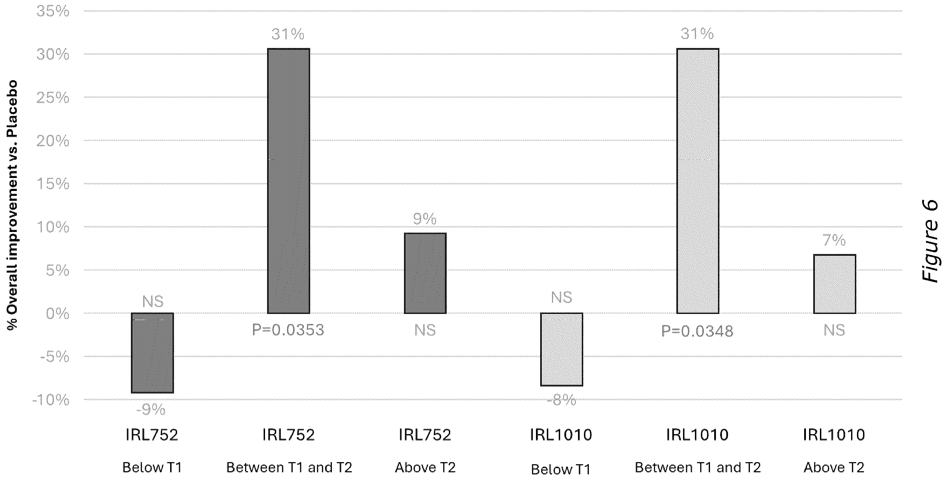

Figure 6 shows the % improvement in fall rate relative to placebo for the three treatment tertiles derived from a negative binomial regression. The T1-T2 tertile showed a 31% relative improvement compared to the placebo over the whole treatment period (12 weeks).

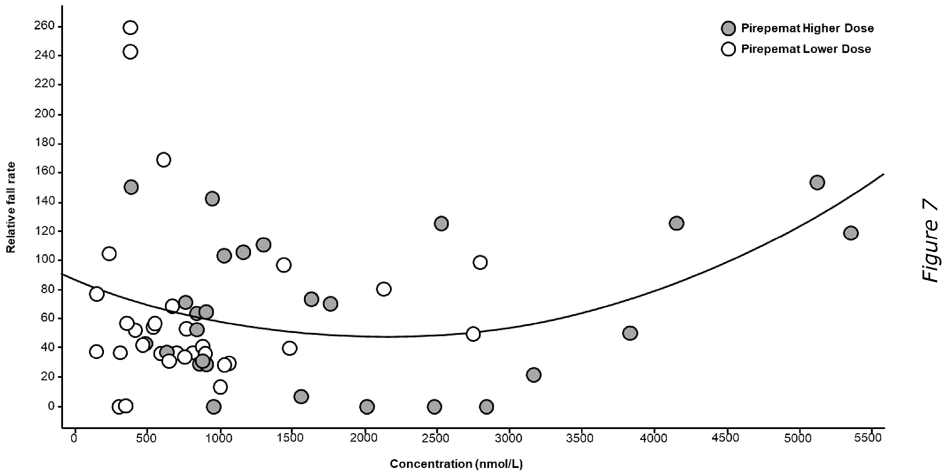

Figure 7 shows the relative fall rate plotted as a function of the minimum (Ctrough) plasma concentration of IRL752 (i.e. pirepemat). It was observed that a plasma concentration of IRL752 from about 500 nmol/L to about 3000 nmol/L provided a reduced relative fall rate. In particular, a plasma concentration from about 500 nmol/L to about 1000 nmol/L of IRL752 provided a reduced relative fall rate.

### EXAMPLES

In this document the naming of the compounds was made using ChemDraw Ultra, version 12.0.2. 1076. If the chemical name and the chemical structure are inconsistent the chemical structure should be considered to be the correct structure.

### Abbreviations

- CAS: Chemical Abstracts Service
- Conc: concentration
- cm: centimeter
- kg: kilogram(s)
- m²: square meter
- mg: milligram(s)
- min: minute(s)
- mol: nanomole(s)
- mL: microliter(s)
- L: liter(s)
- nM: nanoMolar
- M: molar
- MS: Mass Spectometry
- PK: pharmacokinetic
- PK Conc: pharmacokinetic plasma concentration
- SD: standard deviation
- T1: tertial 1
- t.i.d.: ter in die, i.e. three times a day
- UPLC: Ultra-Performance Liquid Chromatography

### Materials and methods

The compound (S)-3-(2,3-difluorophenyl)-3-methoxypyrrolidine, which may also be denominated IRL752, IRL-752 or pirepemat, was provided by Integrative Research Laboratories Sweden AB, Gothenburg, Sweden, as its fumaric acid salt. The molar ratio of (S)-3-(2,3-difluorophenyl)-3-methoxypyrrolidine and fumaric acid in the salt was 1:1. It will be appreciated that all doses or dosages with respect to the fumaric acid salt of IRL752 are calculated on the basis of the compound (S)-3-(2,3-difluorophenyl)-3-methoxypyrrolidine only (i.e. the compound in non-salt form). The study participants were given tablets comprising the fumaric acid salt of (S)-3-(2,3-difluorophenyl)-3-methoxypyrrolidine.

### Clinical study

A clinical study directed to the use of IRL752 for treating falls in patients suffering from Parkinson's disease was performed. 146 study participants were screened of which 104 were randomized to constitute a Full Analysis Set (FAS) population. 90 study participants completed the full study period. The full study period included 6 weeks of screening for falls, followed by a 12 week treatment period after which a follow-up period of 33-37 days took place. The study was performed at 38 clinical sites located in France, Germany, Poland, the Netherlands and Sweden.

During the 6 weeks screening period preceding the treatment period a diary was kept for every study participant in which the number of falls was recorded. A fall was defined as an event, which may include a slip or trip, in which the study participant lost his or her balance and landed on the ground, floor or lower level. This information was subsequently used as a baseline for the 12 week treatment period. For instance, if the study participant fell six times a week during the screening period and this was reduced to three times a week during the treatment period the relative fall rate was 50%.

During the 12 week treatment period oral administration of either placebo or the fumaric acid salt of IRL752 to the study participants took place. The fumaric acid salt of IRL752 was administered orally in a daily dose of 300 mg or 600 mg. The daily dose of 300 mg was provided as three separate doses of 100 mg each. The daily dose of 600 mg was provided as three separate doses of 200 mg each. A clinical assessment of the study participants was conducted at a first baseline visit, after 6 weeks of treatment and after 12 weeks of treatment. The clinical assessment involved *inter alia* measurement of the plasma concentration of IRL752 and/or IRL1010. It will be appreciated that the compound IRL1010 is a metabolite of IRL752 and has been found to have the following chemical structure: 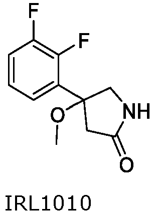 90 study participants completed the study of which 32 study participants were administered a high oral dose of IRL752 (i.e. 600 mg daily), 28 study participants received a low oral dose of IRL752 (i.e. 300 mg daily) and 30 study participants received placebo (i.e. no pharmaceutical drug).

The patient disposition of all patients (i.e. study participants) is shown in Table 1. In Table 1, "N" denotes the total number of study participants. Further, "n" denotes the number of study participants and "(%)" denotes the percentage of study participants. For example, 32 patients out of a total of 35 patients (i.e. 91.4%) completed the study in which IRL752 was administered in a daily dose of 300 mg.

**Table 1: Patient disposition**

| **Disposition** | **IRL752 Daily dose: 300 mg** | **IRL752 Daily dose: 600 mg** | **IRL752 overall** | **Placebo** | **Overall** |
|---|---|---|---|---|---|
| | **N=35 n (%)** | **N=35 n (%)** | **N=70 n (%)** | **N=34 n (%)** | **N=140 n (%)** |
| Screened | | | | | 146 |
| Enrolled | | | | | 106 |
| Randomized | | | | | 104 |
| Screen failure | | | | | 42 |
| Safety Set | 35 (100) | 35 (100) | 70 (100) | 34 (100) | 104 (100) |
| Full Analysis Set | 35 (100) | 33 (94.3) | 68 (97.1) | 33 (97.1) | 101 (97.1) |
| Per-protocol set | 30 (85.7) | 27 (77.1) | 57 (81.4) | 29 (85.3) | 86 (82.7) |
| Completed Trial | 32 (91.4) | 28 (80.0) | 60 (85.7) | 30 (88.2) | 90 (86.5) |
| Patients Discontinuing Study Treatment | 3 (8.6) | 7 (20.0) | 10 (14.3) | 4 (11.8) | 14 (13.5) |

Table 2 shows the demographics and baseline characteristics of the Full Analysis Set of study participants. In Table 2, "N" denotes the number of study participants. Further, "n" denotes the number of study participants and "(%)" denotes the percentage of study participants. As used herein, Safety Set includes all patients who received at least one dose of the investigational medicinal product (i.e. placebo or IRL752). As used herein, Full Analysis Set includes all patients who are randomized and treated, who received at least 1 dose of the investigational medicinal product and provide at least one post baseline efficacy assessment on primary or secondary efficacy endpoints. As used herein, Per Protocol Set includes all patients in the Full Analysis Set who had at least 80% compliance of the study investigational product (recorded compliance between 80% and 120%) and completed Week 11 (Visit 8) of the study with no major protocol deviations that would lead to exclusion from the Per Protocol Set as per decision at blinded data review meeting.

**Table 2: Demographics and Baseline Characteristics**

| **Variable** | **IRL752 Daily dose: 300 mg** | **IRL752 Daily dose: 600 mg** | **IRL752 overall** | **Placebo** | **Overall** |
|---|---|---|---|---|---|
| **Statistic or category** | **N=35 n (%)** | **N=33 n (%)** | **N=68 n (%)** | **N=33 n (%)** | **N=101 n (%)** |
| Age (years) | 71.8 (6.97) | 71.9 (6.61) | 71.8 (6.75) | 71.2 (7.87) | 71.6 (7.10) |
| Mean (SD) | | | | | |
| Sex, n (%) | | | | | |
| Male | 19 (54.3) | 20 (60.6) | 39 (57.4) | 21 (63.6) | 60 (59.4) |
| Female | 16 (45.7) | 13 (39.4) | 29 (42.6) | 12 (36.4) | 41 (40.6) |
| Ethnicity , n % | | | | | |
| White or Caucasian | 34 (97.1) | 33 (100) | 67 (98.5) | 32 (97) | 99 (98.0) |
| Hispanic or Latino | 1 (2.9) | 0 | 1 (1.5) | 1 (3.0) | 2 (2.0) |
| Height, cm | 165.9 | 165.0 | 165.5 | 167.8 | 166.2 |
| Mean(SD) | (9.11) | (9.63) | (9.31) | (10.64) | (9.77) |
| Weight (kg) | 74.4 | 74.7 | 74.6 | 76.8 | 75.3 |
| Mean(SD) | (11.51) | (12.75) | (12.04) | (12.63) | (12.22) |
| Body Mass Index (kg/m²) | 27.1 (4.04) | 27.5 (4.09) | 27.3 (4.04) | 27.4 (4.46) | 27.3 (4.16) |
| Mean(SD) | | | | | |

As used herein, the Body Mass Index (abbreviated BMI) is defined as the body mass divided by the square of the body height and is expressed in units of kg/m².

A statistical analysis was performed which showed that the relative fall rate for study participants having been treated with either a daily dose of 300 mg (provided as 100 mg t.i.d.) or a daily dose of 600 mg (provided as 200 mg t.i.d) of the fumaric acid salt of IRL752 was not significantly different from the relative fall rate observed for study participants having received the treatment using placebo. This is evidenced by Figure 1A and 1B showing the mean fall-rate by treatment group.

### Plasma exposure analysis

An analysis of the plasma concentration of IRL752 and its metabolite IRL1010 in the study participants was performed. The fumaric acid salt of IRL752 was orally administered to the study participants in a daily dosage of 300 mg (i.e. a dosage of 100 mg three times a day, said dosage being administered in the morning, at lunch time and at dinner time) or 600 mg (i.e. a dosage of 200 mg three times a day, said dosage being administered in the morning, at lunch time and at dinner time), and the plasma concentration was measured just before and after two hours of administration of the morning dose after 6 weeks of treatment and also after 11 weeks of treatment. Table 3A shows the plasma concentration morning dose of IRL752 after 6 weeks of treatment just before administration of the morning dose (i.e. the pre-dose value) and two hours after administration of the morning dose (i.e. the 2 hours post-dose value). Table 3B shows the plasma concentration of IRL752 after 11 weeks of treatment just before administration of the morning dose (i.e. the pre-dose value) and two hours after administration of the morning dose (i.e. the 2 hours post-dose value).

**Table 3A: Plasma concentration of IRL752 after 6 weeks**

| **Parameter** | **Pre-dose** | | **2 hours post-dose** | |
|---|---|---|---|---|
| | **IRL752 Daily dose: 300 mg** | **IRL752 Daily dose: 600 mg** | **IRL752 Daily dose: 300 mg** | **IRL752 Daily dose: 600 mg** |
| Number of study participants | 31 | 25 | 32 | 27 |
| Median plasma concentration (nmol/L) | 696 | 2010 | 1890 | 4310 |
| Average plasma concentration (nmol/L) | 960.2 | 2488.44 | 1891.69 | 4330.4 |
| CV% (Coefficient of variation) | 77.2 | 56.3 | 50.9 | 41.3 |

**Table 3B: Plasma concentration of IRL752 after 11 weeks**

| **Parameter** | **Pre-dose** | | **2 hours post-dose** | |
|---|---|---|---|---|
| | **IRL752 Daily dose: 300 mg** | **IRL752 Daily dose: 600 mg** | **IRL752 Daily dose: 300 mg** | **IRL752 Daily dose: 600 mg** |
| Number of study participants | 31 | 25 | 31 | 27 |
| Median plasma concentration (nmol/L) | 756 | 1300 | 2270 | 3720 |
| Average plasma concentration (nmol/L) | 1051.83 | 1927.48 | 2213.84 | 4162.67 |
| CV% (Coefficient of variation) | 86.0 | 75.6 | 42.9 | 54.6 |

Table 4A shows the plasma concentration of IRL1010 after 6 weeks of treatment just before administration of the morning dose (i.e. the pre-dose value) and two hours after administration of the morning dose (i.e. the 2 hours post-dose value). Table 4B shows the plasma concentration of IRL1010 after 11 weeks of treatment just before administration of the morning dose (i.e. the pre-dose value) and two hours after administration of the morning dose (i.e. the 2 hours post-dose value).

**Table 4A: Plasma concentration of IRL1010 after 6 weeks**

| **Parameter** | **Pre-dose** | | **2 hours post-dose** | |
|---|---|---|---|---|
| | **IRL752 Daily dose: 300 mg** | **IRL752 Daily dose: 600 mg** | **IRL752 Daily dose: 300 mg** | **IRL752 Daily dose: 600 mg** |
| Number of study participants | 31 | 25 | 32 | 27 |
| Median plasma concentration (nmol/L) | 959 | 2160 | 1007 | 2180 |
| Average plasma concentration (nmol/L) | 1054.97 | 2347.28 | 1077.09 | 237.9 |
| CV% | 62.5 | 38.5 | 57.3 | 45.2 |

**Table 4B: Plasma concentration of IRL1010 after 11 weeks**

| **Parameter** | **Pre-dose** | | **2 hours post-dose** | |
|---|---|---|---|---|
| | **IRL752** | **IRL752** | **IRL752** | **IRL752** |
| | **Daily dose: 300 mg** | **Daily dose: 600 mg** | **Daily dose: 300 mg** | **Daily dose: 600 mg** |
| Number of study participants | 31 | 25 | 31 | 27 |
| Median plasma concentration (nmol/L) | 1030 | 1840 | 1000 | 1910 |
| Average plasma concentration (nmol/L) | 1161.68 | 2136.16 | 1186.42 | 2222.59 |
| CV% | 72.4 | 41.8 | 65.3 | 41.3 |

It was observed that the plasma concentration of IRL752 varied to a much higher extent than that of IRL1010. For example, after 6 weeks of treatment in a daily dose of 300 mg the pre-dose value of the median plasma concentration of IRL752 was 696 nmol/L which increased to 1890 nmol/L 2 hours after oral administration. In comparison, the plasma concentration of IRL1010 increased from 959 nmol/L to only 1007 nmol/L.

It was concluded that the metabolite IRL1010 has a much longer half-life than IRL752. As a result, the timing of the sampling of e.g. plasma for plasma concentration measurement is less critical for IRL1010 than for IRL752.

It was also found that the plasma concentrations for IRL1010 correlated to the IRL752 plasma concentrations as evidenced by Figure 2. The trough concentration (which may be abbreviated Ctrough) refers to the plasma concentration just before administration of a dose such as a dose in the morning at 6 weeks. The R² value was found to be 0.6568. Further, it was found that plasma concentration of IRL1010 measured 2 hours after administration of IRL752 (i.e. post-dose) correlated to the trough plasma concentration or IRL1010 in the morning at 6 weeks. This is shown in Figure 3B. The R² value was found to be 0.9553. In Figures 2 and 3 each dot represents a study participant.

IRL1010 was also found to have more stable plasma concentrations over 24 hours than IRL752 (suggesting that it has a longer half life), and, was therefore less sensitive to the timing of the dose vs sampling. IRL1010 plasma concentrations also showed smaller intraindividual variability, more accurately reflecting the overall exposure to the active ingredient.

### Impact of plasma concentration of IRL752 and IRL1010 on the mean relative fall rate

In a follow-up assessment, the study participants were divided into three different groups (so-called tertials which are denominated T1, T2 and T3) with 20 study participants in each group based on the plasma concentration of IRL752 as measured for each study participant at 6 and 11 weeks. More specifically, for each study participant given the dosage of 300 mg of IRL752 the plasma concentration of IRL752 was measured just before the morning administration (pre-dose) and also 2 hours after the morning administration of IRL752 (two hours post-dose). The lowest pre-dose concentration value (of the week 6 and week 11 values) was then recorded for IRL752. Thus, in total, 60 plasma concentration values were recorded. The same procedure was performed for the dosage of 600 mg of IRL752. The plasma concentration values for the study participants were then divided into three tertial concentration ranges,

Three tertial concentration ranges were identified, namely "Below T1 PK Conc", "T1 to T2 PK Conc" and "Above T2 PK Conc". Further, a "Zero PK Conc" range was found for the placebo. The results are shown in Table 5. The same procedure was repeated, but the analysis was made with respect to the plasma concentration of IRL1010 instead of IRL752. The results are shown in Table 6.

**Table 5: Plasma concentration ranges for IRL752**

| | **Below T1 PK Conc, i.e. from 0 to about 632 nmol/l** | **T1 to T2 PK Conc, i.e. from about 632 to about 1060 nmol/L** | **Above T2 PK Conc, i.e. from about 1060 to about 5350 nmol/L** | **Zero PK Conc, i.e. equal to 0 nmol/L** | **Total number of study participants** |
|---|---|---|---|---|---|
| IRL752, Daily dose: 600 mg | 2 | 7 | 18 | 0 | 27 |
| IRL752, Daily dose: 300 mg | 18 | 13 | 1 | 0 | 32 |
| Placebo | 0 | 0 | 0 | 30 | 30 |
| Total number of study participants | 20 | 20 | 19 | 30 | 89 |
| Missing PK | | | | | 4 |

**Table 5: Plasma concentration ranges for IRL1010**

| | **Below T1 PK Conc, i.e. from 0 to about 845 nmol/l** | **T1 to T2 PK Conc, i.e. from about 845 to about 1540 nmol/L** | **Above T2 PK Conc, i.e. from about 1540 to about 3990 nmol/L** | **Zero PK Conc, i.e. equal to 0 nmol/L** | **Total number of study participants** |
|---|---|---|---|---|---|
| IRL752, Daily dose: 600 mg | 3 | 10 | 15 | 0 | 27 |
| IRL752, Daily dose: 300 mg | 17 | 10 | 5 | 0 | 32 |
| Placebo | 0 | 0 | 0 | 30 | 30 |
| Total number of study participants | 20 | 20 | 19 | 30 | 89 |
| Missing PK | | | | | 4 |

Further, the mean relative fall rate for the three plasma concentration values for IRL752 and placebo were plotted to provide Figure 4A showing that the concentration "T1 to T2 PK Conc", i.e. a plasma concentration of about 632 to about 1060 nmol/L of IRL752, provides a significant reduction of the fall rate as compared to placebo. In contrast, the plasma concentrations "Below T1 PK Conc", i.e. from 0 to about 632 nmol/l, and "Above T2 PK Conc", i.e. from about 1060 to about 5350 nmol/L, did not provide a significant reduction of the fall rate as compared to placebo. The mean relative fall rate by plasma concentration tertials is also illustrated in Figure 4B showing that the mid tertile group, in which the study participants had a plasma concentration of about 632 to about 1060 nmol/L of IRL752, had a significantly lower mean fall rate.

In the same way, Figure 5 shows the minimum (Ctrough) plasma concentration values for IRL1010 and placebo, and that the concentration "T1 to T2 1010 Conc", i.e. a plasma concentration from about 845 to 1540 nmol/L of IRL1010, provides a significant reduction of the fall rate as compared to placebo. In contrast, the plasma concentrations "Below T1 PK Conc", i.e. from 0 to about 845 nmol/l, and "Above T2 PK Conc", i.e. from about 1540 to about 3990 nmol/L, did not provide a significant reduction of the fall rate as compared to placebo.

More specifically, the overall improvement of the (relative) fall rate based on IRL752 or IRL1010 was found to be 31% as compared to placebo for the minimum plasma concentration (Ctrough) "T1 to T2 1010 Conc" as shown in Figure 6. Figure 6 also shows that the reduction in (relative) fall rate for the plasma concentrations "Below T1 PK Conc" and "Above T2 PK Conc" was not significant (denominated NS in Figure 6).

Thus, neither the highest plasma concentration (i.e. "Above T2 PK Conc") nor the lowest plasma concentration (i.e. "Below T1 PK Conc") resulted in a relative fall rate that was significantly different from the relative fall rate observed for study participants having received the treatment using placebo. Unexpectedly, the plasma concentration between the highest and lowest plasma concentration (i.e. T1 to T2 PK Conc) was instead found to result in a relative fall rate that was significantly improved, i.e. lowered, compared to the relative fall rate observed for study participants having received the treatment using placebo.

The relative fall rate was then plotted as a function of the plasma concentration of IRL752 as shown in Figure 7. IRL752 (i.e. pirepemat) fumaric acid salt was provided in a lower dose (i.e. a daily dose of 300 mg) or in a higher dose (i.e a daily dose of 600 mg).

It was concluded that a reduced fall rate such as a reduced relative fall rate can be achieved when the plasma concentration of IRL752 of a patient such as a human is from about 500 nmol/L to about 3000 nmol/L, such as from about 500 nmol/L to about 2000 nmol/L, such as from about 500 nmol/L to about 1000 nmol/L. It will be appreciated that this may correspond to a plasma concentration range of IRL1010 of from about 800 nmol/L to about 3200 nmol/L, such as from about 800 nmol/L to about 2400 nmol/L, such as from about 800 nmol/L to about 1600 nmol/L.

### Testing for biphasic behaviour

To test for a biphasic response, i.e. the relationship between fall rates during the study and plasma concentration of IRL752, a negative binomial regression model with log (fall rate at baseline), days during baseline period and linear and quadratic terms for IRL752 concentration use as covariates and log(time) during the treatment period as the offset parameter, was fitted, using the lower of the two trough values for IRL752 plasma concentration measured at visit 5 and visit 8.

This model finds a negative value for the coefficient corresponding to the linear component (p = 0.031) and a positive value for the quadratic component (p = 0.024), thus demonstrating a biphasic concentration-response relationship As shown herein, the plasma concentration of about 632 to about 1060 nmol/L of IRL752 provided a reduced fall rate while plasma concentrations outside this range did not result in a reduced fall rate. Thus, the effect of reduced fall rate is associated with a specific plasma concentration and consequently with the dosage providing said specific plasma concentration. Only a dosage of IRL providing a plasma concentration of about 632 to about 1060 nmol/L of IRL752 allows for a reduction in fall rate such as mean relative fall rate.

### Determination of the concentration of the compounds IRL752, IRL991 and IRL1010 in human plasma by UPLC-MS/MS

The concentration in human plasma of the compound IRL752 as described herein and its metabolites IRL991 and IRL1010 was performed as described below.

### Chemicals

IRL752 (reference material, MW 213.2 g/mol)
IRL991 (reference material, MW 213.2 g/mol)
IRL1010 (reference material, MW 227.2 g/mol)
IRL921 (reference material, IS for IRL752 MW 216.3 g/mol)
IRL1059 (reference material, IS for IRL991 and IRL1010, MW 230.2 g/mol)
Water purified in a MilliQ Purification System (MQ)
Acetonitrile CAS 75-05-8, p.a (ACN)
Formic acid CAS 64-18-6, p.a (FA)

### Chemical naming and structures

Below the chemical compounds have been depicted as racemates. However, the skilled person understands that the compounds may be provided as an enantiomer. For instance, IRL752 may be an (S)-enantiomer as described in this document. Further, it will be understood that IRL1010 is likely to be provided as an (S)-enantiomer when it is generated by from the (S)-enantiomer of IRL752 in blood plasma.

| | |
|---|---|
| IRL752 | 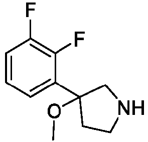 |
| IRL991 | 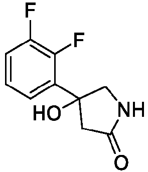 |
| IRL1010 | 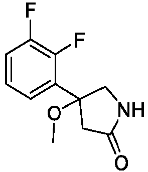 |
| IRL921 | 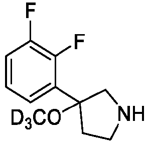 |
| IRL1059 | 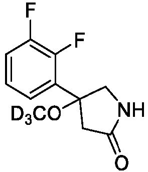 |

### Equipment

### UPLC-MS/MS equipment Acquity Xevo TQSµ

Waters Acquity UPLC
Column: Acquity UPLC BEH C18 (50 x 2.1 mm, 1.7 µm)
Column temperature: 60 °C
Sample temperature: 10°C.

### Waters Xevo TQSµ

Ionization method: Electrospray, positive mode
Data acquisition: Multiple Reaction Monitoring (MRM)
Software: MassLynx V4.1SCN945SCN960
Software: UNIFI version 1.8

### Chromatographic conditions Waters Acquity

### Mobile phase:

Solution A: 0.1% formic acid in water
Solution B: 0.1% formic acid in acetonitrile
Flow-rate: 600 µL/min
Injection volume: 1 µL
Sample Manager Wash: Acetonitrile: Water (50:50) with 0.1%FA
Sample Manager Purge: 0.1% formic acid in water

### Gradient program:

| Time | A% | B | %Flow rate |
|---|---|---|---|
| 0.0 min | 98 | 2 | 600 mL/min |
| 1.5 min | 5 | 95 | 600 mL/min |
| 1.8 min | 5 | 95 | 600 mL/min |
| 1.85 min | 98 | 2 | 600 mL/min |
| 2.0 min | 98 | 2 | 600 mL/min |

Total run time: 2.0 minutes

### Other equipment

Finnpipett 0.5-10 µL, 5-50 µL, 20-200 µL, 100-1000 µL, 1-5 mL
Biohit Picus 10-300 µL, 50-1000 µL, 100-5000 µL
Biohit e300, 8 channel pipett
VWR DVX-2500 Digital multitube vortexer
Centrifuge: SIGMA Laboratory Centrifuges 4K15 371/372
Analytical balance: Sartorius ME 235P

### Mass spectrometric conditions for Xevo TQ-Sµ

Tuning of the interfacial parameters, collision energy and collision gas (argon) pressure shall be carried out. The tuning procedure shall be focused on sensitivity optimization for the MRM transition m/z:

| | |
|---|---|
| 214 > 182 | IRL752 |
| 217 > 182 | IRL921 (IS for IRL752) |
| 214 > 154 | IRL991 |
| 228 > 196 | IRL1010 |
| 231 > 196 | IRL1059 (IS for IRL1010 and IRL991 |

### Mobile Phase A: 0.1% formic acid in water

Prepare a solution of 0.1% formic acid in water. For example, add 1 mL formic acid into a 1000 mL flask. Dilute to 1000 mL with MilliQ-water and mix well.

### Mobile Phase B: 0.1% formic acid in acetonitrile

Prepare a solution of 0.1% formic acid in acetonitrile. For example, add 1 mL formic acid into a 1000 mL flask. Dilute to 1000 mL with acetonitrile and mix well.

### Preparation of reference stock solutions

### Check the analytical balance in accordance with the instruction.

Weigh approximately 4 mg of each reference substance IRL752 (fumarate salt), IRL991 and IRL1010 in 5 mL volumetric flasks, dissolve with ACN:MQ (1:1) and dilute with the same solvent to the mark of the volumetric flask.

### Print a report from the weighing and sign it.

The concentrations of the reference stock solution will be approximately 0.80 mg/mL of each reference substance respectively, depending on the exact amount of substance that has been weighed. The exact amount is given on the printed report.

Two weightings of this kind for each of the three reference substances are to be made. These are denoted:
Stock_1 (marked with GLP number, concentration of IRL752, preparation date and signature)
Stock_2 (marked with GLP number, concentration of IRL752, preparation date and signature)
Stock_3 (marked with GLP number, concentration of IRL991, preparation date and signature)
Stock_4 (marked with GLP number, concentration of IRL991, preparation date and signature)
Stock_5 (marked with GLP number, concentration of IRL1010, preparation date and signature)
Stock_6 (marked with GLP number, concentration of IRL1010, preparation date and signature)
Store at -20 °C.

### Preparation of calibration and QC sample stock solutions and diluted stock solutions

All calibration sample spiking solutions are prepared from the stock solution Stock_1, Stock 3 and Stock 5 and all QC sample spiking solutions are prepared from the stock solution Stock_2. Stock 4 and Stock 6. Calibration sample spiking solutions and QC sample spiking solutions are diluted with ACN:MQ (1:1).

Std Mix_1 and QC Mix_1: Prepare a solution that corresponds to a concentration of approximately 200 µM of IRL752.

For example, if the stock solution (Stock_1) contains 0.5 mg/mL, 0.825 mL of Stock_1, is diluted to 10 mL with ACN:MQ (1:1) ≈ 200 µM IRL752.

Std Mix_2: 1 mL of Std Mix_1 is diluted to 10 mL with ACN:MQ (1:1)≈ 20 µM IRL752. Std Mix_3: 1 mL of Std Mix_2 is diluted to 10 mL with ACN:MQ (1:1)≈ 2.0 µM IRL752.

QC Mix_2: 1 mL of QC Mix_1 is diluted to 10 mL with ACN:MQ (1:1)≈ 20 µM IRL752. QC Mix_3: 1 mL of QC Mix_2 is diluted to 10 mL with ACN:MQ (1:1)≈ 2.0 µM IRL752. Std Mix_4 and QC Mix_4: Prepare a solution that corresponds to a concentration of approximately 400 µM of IRL991 and 200 µM IRL1010.

For example, if both the stock solution for IRL991 (Stock_3) and stock solution for IRL1010 (Stock 5) contains 0.50 mg/mL, 1.068 mL of Stock_3 and 0.592 mL of Stock 5, is diluted to 10 mL with ACN:MQ (1:1) ≈ 400 µM IRL991 and ≈ 200 µM IRL1010 respectively.

Std Mix_5: 1 mL of Std Mix_4 is diluted to 10 mL with ACN:MQ (1:1)≈ 40 µM IRL991 and ≈ 20 µM IRL1010.

Std Mix_6: 1 mL of Std Mix_5 is diluted to 10 mL with ACN:MQ (1:1)≈ 4 µM IRL991 and ≈ 2 µM IRL1010.

QC Mix_5: 1 mL of QC Mix_4 is diluted to 10 mL with ACN:MQ (1:1)≈ 40 µM IRL991 and ≈ 20 µM IRL1010.

QC Mix_6: 1 mL of QC Mix_5 is diluted to 10 mL with ACN:MQ (1:1)≈ 4 µM IRL991 and ≈ 2 µM IRL1010.

The stock solutions are transferred to centrifuge tubes. Store at -20°C.

These are marked with:
Std Mix_1, Std Mix_2, Std Mix_3, Std Mix_4, Std Mix_5, Std Mix_6, QC
Mix_1, QC Mix_2, QC Mix_3, QC Mix_4, QC Mix_5, QC Mix_6,
GLP number, preparation date and signature.

### Preparation of calibration sample spiking solutions

All calibration samples contain all three analytes and are diluted with ACN:MQ (1:1) to desired concentration, see below.

| IRL752 | IRL991 | IRL1010 |
|---|---|---|
| Cal 1: 12.0 nM | Cal 1: 30.0 nM | Cal 1: 15.0 nM |
| Cal 2: 36.0 nM | Cal 2: 60.0 nM | Cal 2: 30.0 nM |
| Cal 3: 75.0 nM | Cal 3: 120 nM | Cal 3: 60.0 nM |
| Cal 4: 200 nM | Cal 4: 400 nM | Cal 4: 200 nM |
| Cal 5: 800 nM | Cal 5: 1000 nM | Cal 5: 500 nM |
| Cal 6: 2000 nM | Cal 6: 4000 nM | Cal 6: 2000 nM |
| Cal 7: 8000 nM | Cal 7: 12000nM | Cal 7: 6000 nM |
| Cal 8: 12000 nM | Cal 8: 22000 nM | Cal 8: 11000 nM |

Cap the tubes and store at -20°C.

### Preparation of QC sample spiking solutions

All calibration samples are diluted with ACN:MQ (1:1) to desired concentration, see below.

| IRL752 | IRL991 | IRL1010 |
|---|---|---|
| LLOQ: 12.0 nM | LLOQ: 30.0 nM | LLOQ: 15.0 nM |
| QCL: 36.0 nM | QCL: 60.0 nM | QCL: 30.0 nM |
| QCM: 1000 nM | QCM: 800 nM | QCM: 400 nM |
| QCH: 7000 nM | QCH: 10000 nM | QCH: 5000 nM |

Cap the tube and store at -20°C.

### Preparation of internal standard solutions

Weigh approximately 1 mg of IRL921 (oxalate salt, IS for IRL752) in 5 mL volumetric flasks, dissolve with ACN:MQ (1:1) and dilute with the same solvent to the mark of the volumetric flask (Stock 7). Weigh approximately 1 mg of IRL1059 (IS for IRL991 and IRL1010) in 5 mL volumetric flasks, dissolve with ACN:MQ (1:1) and dilute with the same solvent to the mark of the volumetric flask Stock 8.

Stock solution Stock_7: Approximately 0.20 mg/mL IRL921 (oxalate salt), depending on the exact amount of substance that has been weighed.

Stock solution Stock_8: Approximately 0.20 mg/mL IRL1059, depending on the exact amount of substance that has been weighed.

These are denoted:
Stock_7 (marked with GLP number, preparation date and signature)
Stock_8 (marked with GLP number, preparation date and signature)
Store at -20°C.

### Protein perception solution (PPS-IS):

Approximately 12 nM IRL921 and 6 nM IRL1059.

IS Mix_1: Prepare a solution that corresponds to a concentration of approximately 10.0 µM of IRL921.

IS Mix_2: Prepare a solution that corresponds to a concentration of approximately 10.0 µM of IRL1059.

For example, if the stock solution (Stock_7) contains 1.0 mg/mL, 0.154 mL of Stock_7, is diluted to 10 mL with ACN:MQ (1:1) ≈ 10.0 µM IRL921.

PPS-IS solution: Prepare a solution corresponding to a concentration of 12.1 nM IRL921 and 6 nM IRL1059. For example, add 0.6 mL IS Mix_1 and 0,6 mL of IS Mix_2 to a 500 mL volumetric flask and dilute to the mark with acetonitrile. Mix well. Other volumes can be prepared if needed.

Store at 4-8°C.

### Control of the equipment

For the pipettes, centrifuge, analytical balance, UPLC-MS/MS see the respective apparatus manual.

### Control of method

At least 6 QC samples spread at 3 different levels for IRL752, IRL991 and IRL1010 shall be run together with each analytical batch.

### Calibration of method

Two sets of calibration samples at 8 different levels for IRL752, IRL991 and IRL1010 shall be run together with each analytical batch. See "Preparation of calibration sample spiking solutions".
Calibration For the pipettes, centrifuge, balance, UPLC-MS/MS see the respective apparatus manual

### Analytical procedure

### Sample treatment for the plasma Blank, Calibration, QC and Study samples

Thaw all samples and let them equilibrate to room temperature.

Vortex mix and centrifuge all samples.

### Pretreatment of calibration samples

100 µL of blank human Li- heparin plasma is added into a 2.0 mL 96-well plate and spiked with 100 µL of the calibration sample spiking solution. 600 µL of PPS-IS solution is added.

### Pretreatment of QC samples

100 µL of blank human Li-heparin plasma is added into the 2.0 mL 96 well plate and spiked with 100 µL of the QC sample spiking solution. 600 µL of PPS-IS solution is added.

### Pretreatment of blank plasma sample with internal standard

100 µL of blank human Li heparin plasma is added into the 96 well plate. 100 µL ACN:MQ (1:1) and 600 µL of PPS-IS solution is added.

### Pretreatment of blank plasma sample without internal standard

100 µL of blank human Li heparin plasma is added into the 96 well plate. 100 µL ACN:MQ (1:1) and 600 µL acetonitrile is added.

### Pretreatment of study samples

100 µL of study sample plasma is added into the 96 well plate. 100 µL ACN:MQ (1:1) and 600 µL of PPS-IS solution is added.

### Further pretreatment of all samples

### Cap the 96 well plate and vortex mix for 10 min (pulse1200).

Centrifuge the samples for 10 minutes at 2862 RCF (4000 rpm). Inject 1 µL into the UPLC-system.

### Quality assurance Acceptance of an analytical batch

Injection order of the samples in a batch are:
Blank solvent(s)
Plasma blank
Plasma blank with IS
Calibration samples
Blank solvent(s)
QC samples
Blank solvent(s)
Study samples
Blank solvent(s)
QC samples
Blank solvent(s)
Plasma blank
Plasma blank with IS
Calibration samples
Blank solvent(s)

Each calibration interval shall have determined concentrations within ±15 % (20% for the lowest calibration point), when back calculated using the regression equation, with at least 12 of 16 (75%) for IRL752, IRL991 and IRL1010 respectively of the calibration levels being included in the calibration curve. The lowest and highest included calibration sample will determine the calibration interval for the analytical batch.

At least four out of six QC samples shall be within ±15 % of their respective nominal values, when back calculated using the regression equation and at least one QC of each level shall be within the acceptance criteria. Single wells can be re-injected or single samples can be reanalyzed even though the analytical batch has been accepted if problems have arisen (for example instrumental problems, broken tubes or vials etc.). All reanalyzes will be noted and fully motivated in the laboratory workbook.

### Calculations

The calibration curves are constructed as the chromatographic peak area ratio (analyte/internal standard) as a function of analyte concentration. Linear regression with the weighting factor of 1/x2 for IRL752, IRL991 and IRL1010 is used for curve fitting. The concentrations of IRL752 in the study samples and QC samples are calculated using the linear regression equation obtained.

### Interval

IRL752: 12.0 - 12000 nM in human plasma.
IRL991: 30.0-22000 nM in human plasma.
IRL1010: 15.0-11000 nM in human plasma.

### Lowest limits of quantification (LLOO)

IRL752: 12.0 nM using 100 µL sample volume.
IRL991: 30.0 nM using 100 µL sample volume.
IRL1010: 15.0 nM using 100 µL sample volume.

### Determination of half lives of IRL752, IRL991 and IRL1010

In a pharmacokinetic study in elderly humans (200 mg of IRL752 t.i.d.), described in Erik Rein-Hedin et al. First-in-Human Study to Assess the Safety, Tolerability, and Pharmacokinetics of Pirepemat, a Cortical Enhancer, in Healthy Volunteers, Clinical Pharmacology in Drug Development, 2021, 10(12), 1485-1494 (https://doi.ora/10.1002/cpdd.959) the following half-lives were determined for IRL752, IRL1010 and IRL991.
t1/2 (IRL752): 5.85 h
t1/2 (IRL1010): 8.68 h
t1/2 (IRL991): 23.3 h

## Claims

1. The compound (S)-3-(2,3-difluorophenyl)-3-methoxypyrrolidine, or a pharmaceutically acceptable salt thereof, 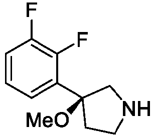 for use in the treatment of a disorder associated with a neurodegenerative disease, wherein the treatment comprises administering (S)-3-(2,3-difluorophenyl)-3-methoxypyrrolidine, or a pharmaceutically acceptable salt thereof, to a subject in an amount that results in:
(i) a minimum plasma concentration of (S)-3-(2,3-difluorophenyl)-3-methoxypyrrolidine of from about 500 nM to about 3000 nM before dosing in the morning; and/or
(ii) a minimum plasma concentration of 4-(2,3-difluorophenyl)-4-methoxy-2-pyrrolidinone 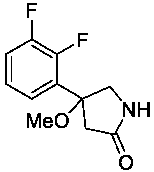 of about 800 nM to about 3200 nM before dosing in the morning.

2. The compound for use as claimed in Claim 1, wherein the treatment comprises the steps of:
(i) determining the dose of (S)-3-(2,3-difluorophenyl)-3-methoxypyrrolidine required to result in a minimum plasma concentration of (S)-3-(2,3-difluorophenyl)-3-methoxypyrrolidine and/or 4-(2,3-difluorophenyl)-4-methoxy-2-pyrrolidinone as defined in Claim 1; then
(ii) proceeding with the treatment using the dose so determined.

3. The compound for use as claimed in Claim 1 or Claim 2, wherein the amount of (S)-3-(2,3-difluorophenyl)-3-methoxypyrrolidine, or a pharmaceutically acceptable salt thereof, required to result in the minimum plasma concentrations of (S)-3-(2,3-difluorophenyl)-3-methoxypyrrolidine and/or 4-(2,3-difluorophenyl)-4-methoxy-2-pyrrolidinone, defined in Claim 1 is determined by titration.

4. The compound for use as claimed in Claim 3, wherein the titration comprises the steps of:
(i) administering a first dose of (S)-3-(2,3-difluorophenyl)-3-methoxypyrrolidine, or a pharmaceutically acceptable salt thereof, of from about 25 mg to about 100 mg (of the active ingredient), 1 to 3 times a day, to a subject during a first time period sufficient to achieve a steady state plasma concentration of (S)-3-(2,3-difluorophenyl)-3-methoxypyrrolidine, 4-(2,3-difluorophenyl)-4-methoxy-2-pyrrolidinone and/or 4-(2,3-difluorophenyl)-4-hydroxy-2-pyrrolidinone optionally, wherein the time period is from about 1 day to about 10 days;
(ii) measuring the minimum plasma concentration of (S)-3-(2,3-difluorophenyl)-3-methoxypyrrolidine, 4-(2,3-difluorophenyl)-4-methoxy-2-pyrrolidinone and/or 4-(2,3-difluorophenyl)-4-hydroxy-2-pyrrolidinone; and
(iii)determining the dose of (S)-3-(2,3-difluorophenyl)-3-methoxypyrrolidine, or a pharmaceutically acceptable salt thereof, required to provide a minimum plasma concentration within the ranges for (S)-3-(2,3-difluorophenyl)-3-methoxypyrrolidine and/or 4-(2,3-difluorophenyl)-4-methoxy-2-pyrrolidinone defined in Claim 1.

5. The compound for use as claimed in Claim 3, wherein step (iii) comprises the steps of:
(iv)administering an increased dose of (S)-3-(2,3-difluorophenyl)-3-methoxypyrrolidine, or a pharmaceutically acceptable salt thereof, for a further time period, which dose is from about 25 to about 100 mg (of the active ingredient) higher than the previous dose, 1 to 3 times a day, to the subject for a time period sufficient to achieve a steady state plasma concentration of (S)-3-(2,3-difluorophenyl)-3-methoxypyrrolidine and/or 4-(2,3-difluorophenyl)-4-methoxy-2-pyrrolidinone, optionally wherein the time period is from about 1 day to about 10 days;
(v) measuring the minimum plasma concentration of (S)-3-(2,3-difluorophenyl)-3-methoxypyrrolidine, 4-(2,3-difluorophenyl)-4-methoxy-2-pyrrolidinone and/or 4-(2,3-difluorophenyl)-4-hydroxy-2-pyrrolidinone; and
(vi) optionally, repeating steps (iv) and (v), until a minimum plasma concentration within the ranges for (S)-3-(2,3-difluorophenyl)-3-methoxypyrrolidine and/or 4-(2,3-difluorophenyl)-4-methoxy-2-pyrrolidinone defined in Claim 1 is achieved.

6. The compound for use as claimed in Claim 4 or Claim 5, wherein step (iii) comprises calculating the dose required to provide a minimum plasma concentration within the ranges for (S)-3-(2,3-difluorophenyl)-3-methoxypyrrolidine and/or 4-(2,3-difluorophenyl)-4-methoxy-2-pyrrolidinone defined in Claim 1 from the minimum plasma concentrations measured in step (ii) and, optionally, step (v).

7. The compound for use as claimed in any one of Claims 3 to 6, wherein the minimum plasma concentration of (S)-3-(2,3-difluorophenyl)-3-methoxypyrrolidine and/or 4-(2,3-difluorophenyl)-4-methoxy-2-pyrrolidinone is measured in step (ii) and step (v).

8. The compound for use as claimed in any one of Claims 2 to 7, wherein the minimum plasma concentration of 4-(2,3-difluorophenyl)-4-methoxy-2-pyrrolidinone is used to determine the dose of (S)-3-(2,3-difluorophenyl)-3-methoxypyrrolidine, or a pharmaceutically acceptable salt thereof.

9. The compound for use as claimed in any one of Claims 1 to 8, wherein the compound is in the form of a fumarate salt.

10. The compound for use as claimed in any one of Claims 1 to 9, wherein the neurogenerative disease is Parkinson's disease or Alzheimer's disease, optionally wherein the neurodegenerative disease is Parkinson's disease.

11. The compound for use as claimed in any one of Claims 1 to 10, wherein the disorder associated with a neurodegenerative disease is a cognitive disorder or a movement disorder; optionally wherein the disorder associated with a neurodegenerative disease is a movement disorder, such as
postural instability.

12. The compound for use as claimed in Claim 11, wherein the treatment of postural instability comprises reducing the frequency of falls experienced by the subject.

13. The compound for use as claimed in any one of Claims 1 to 12, wherein the minimum plasma concentration of (S)-3-(2,3-difluorophenyl)-3-methoxypyrrolidine before dosing in the morning is from about 500 nM to about 2000 nM, optionally wherein the minimum plasma concentration of (S)-3-(2,3-difluorophenyl)-3-methoxypyrrolidine before dosing in the morning is from about 500 nM to about 1000 nM; and/or
the minimum plasma concentration of 4-(2,3-difluorophenyl)-4-methoxy-2-pyrrolidinone before dosing in the morning is from about 800 nM to about 2400 nM, optionally wherein the minimum plasma concentration of 4-(2,3-difluorophenyl)-4-methoxy-2-pyrrolidinone before dosing in the morning is from about 800 nM to about 1600 nM.

14. The compound for use as claimed in any one of Claims 1 to 13, wherein the 4-(2,3-difluorophenyl)-4-methoxy-2-pyrrolidinone is (S)-4-(2,3-difluorophenyl)-4-methoxy-2-pyrrolidinone.

15. The compound for use as claimed in any one of Claims 1 to 14, wherein the plasma concentration of (S)-3-(2,3-difluorophenyl)-3-methoxypyrrolidine and 4-(2,3-difluorophenyl)-4-methoxy-2-pyrrolidinone (including(S)-4-(2,3-difluorophenyl)-4-methoxy-2-pyrrolidinone) is measured by liquid chromatography, optionally wherein the plasma concentration is measured by liquid chromatography with tandem mass spectrometry (LC-MS/MS).

16. The compound for use as claimed in any one of Claims 1 to 15, wherein the minimum plasma concentration before dosing in the morning is Cmin or Ctrough, optionally Ctrough.

17. The compound for use as claimed in any one of Claims 1 or 9 to 15, wherein the treatment comprises determining the dose of (S)-3-(2,3-difluorophenyl)-3-methoxypyrrolidine required to result in the plasma concentration of (S)-3-(2,3-difluorophenyl)-3-methoxypyrrolidine and/or 4-(2,3-difluorophenyl)-4-methoxy-2-pyrrolidinone as defined in any one of Claims 1 to 15, comprising the steps of:
(a) administering (S)-3-(2,3-difluorophenyl)-3-methoxypyrrolidine, or a pharmaceutically acceptable salt thereof, to the subject;
(b) measuring the plasma concentration of (S)-3-(2,3-difluorophenyl)-3-methoxypyrrolidine and/or 4-(2,3-difluorophenyl)-4-methoxy-2-pyrrolidinone in the subject;
(c) comparing the plasma concentrations of the (S)-3-(2,3-difluorophenyl)-3-methoxypyrrolidine and/or 4-(2,3-difluorophenyl)-4-methoxy-2-pyrrolidinone to the concentrations defined in Claim 1 or Claim 13
(d) determining whether the plasma concentration of (S)-3-(2,3-difluorophenyl)-3-methoxypyrrolidine and/or 4-(2,3-difluorophenyl)-4-methoxy-2-pyrrolidinone (e.g. (S)-4-(2,3-difluorophenyl)-4-methoxy-2-pyrrolidinone) is within the reference range for the treatment of a disease associated with a neurodegenerative disease and related disorders; and, if not, adjusting the dose of (S)-3-(2,3-difluorophenyl)-3-methoxypyrrolidine administered to the subject to provide a dose that results in a plasma concentration within the effective range for the subject being treated.

18. A compound, or a pharmaceutically acceptable salt or deuterated variant thereof, selected from the group consisting of:
4-(2,3-difluorophenyl)-4-methoxy-2-pyrrolidinone 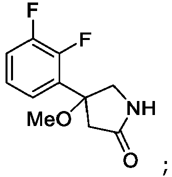
(*S*)-4-(2,3-difluorophenyl)*-*4-methoxy-2*-*pyrrolidinone 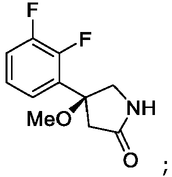
4-(2,3-difluorophenyl)-4-hydroxy-2-pyrrolidinone 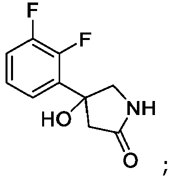 and
(*S*)*-*4-(2,3-difluorophenyl)-4-hydroxy-2-pyrrolidinone

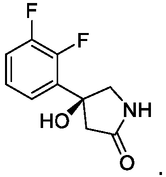

19. The compound for use as claimed in any one of Claim 17wherein the minimum plasma concentration of 4-(2,3-difluorophenyl)-4-methoxy-2-pyrrolidinone, optionally (S)-4-(2,3-difluorophenyl)-4-methoxy-2-pyrrolidinone, is used to determine the necessary dose of (S)-3-(2,3-difluorophenyl)-3-methoxypyrrolidine.

20. A method of determining an effective dose of (S)-3-(2,3-difluorophenyl)-3-methoxypyrrolidine, or a pharmaceutically acceptable salt thereof, for the treatment of a disorder associated with a neurodegenerative disease, comprising the steps of:
(a) administering the compound (S)-3-(2,3-difluorophenyl)-3-methoxypyrrolidine, or a pharmaceutically acceptable salt thereof, to a subject;
(b) obtaining a plasma sample from the subject;
(c) measuring the concentration of one or more metabolites of (S)-3-(2,3-difluorophenyl)-3-methoxypyrrolidine, selected from the group consisting of:
4-(2,3-difluorophenyl)-4-methoxy-2-pyrrolidinone;
(*S*)-4-(2,3-difluorophenyl)-4-methoxy-2-pyrrolidinone;
4-(2,3-difluorophenyl)-4-hydroxy-2-pyrrolidinone; and
(*S*)-4-(2,3-difluorophenyl)-4-hydroxy-2-pyrrolidinone in the plasma sample
(d) comparing the plasma concentration of the one or more metabolites to a reference value; and
(e) determining an effective dose of (S)-3-(2,3-difluorophenyl)-3-methoxypyrrolidine for the treatment of a disorder associated with a neurodegenerative disease.
